# EUROPEAN PATENT SPECIFICATION

(11) **EP 3 257 950 B1**
(45) Date of publication and mention of the grant of the patent: **08.04.2020**
(21) Application number: 17170466.1
(22) Date of filing: 28.09.2011
(51) Int. Cl.: C12Q 1/6886

(54) **METHODS AND MEANS FOR TYPING A SAMPLE COMPRISING CANCER CELLS BASED ON ONCOGENIC SIGNAL TRANSDUCTION PATHWAYS**
VERFAHREN UND MITTEL ZUR TYPISIERUNG PROBE MIT KREBSZELLEN AUF BASIS VON ONKOGENEN SIGNALTRANSDUKTIONSPFADEN
PROCÉDÉS ET MOYENS DE TYPAGE D'UN ÉCHANTILLON COMPRENANT DES CELLULES CANCÉREUSES BASÉS SUR LES VOIES DE TRANSDUCTION DU SIGNAL ONCOGÈNE

(30) Priority: 28.09.2010 WO PCT/NL2010/050634
(43) Date of publication of application: 20.12.2017
(62) Divisional of application: 11791635.3
(73) Proprietor: Agendia N.V., 1098 XH Amsterdam (NL)
(72) Inventor: Sun, Tian, 1363 BB Almere (NL); Roepman, Paul, 3723 RA Bilthoven (NL); Glas, Annuska Maria, 1567 JH Assendelft (NL); Bernards, Rene, 1391 AD Abcoude (NL); Simon, Iris, 1054 BT Amsterdam (NL)
(74) Representative: V.O.

(56) References cited:
- EP-A1- 2 202 320
- WO-A1-2009/140409
- ITADANI HIRAKU ET AL: "Can systems biology understand pathway activation? Gene expression signatures as surrogate markers for understanding the complexity of pathway activation", CURRENT GENOMICS, BENTHAM SCIENCE PUBLISHERS LTD, NL, vol. 9, no. 5, 1 August 2008 (2008-08-01), pages 349-360, XP009116688, ISSN: 1389-2029, DOI: DOI:10.2174/138920208785133235
- KIM IL-JIN ET AL: "Oligonucleotide microarray analysis of distinct gene expression patterns in colorectal cancer tissues harboring BRAF and K-ras mutations", CARCINOGENESIS, OXFORD UNIVERSITY PRESS, GB, vol. 27, no. 3, 1 March 2006 (2006-03-01), pages 392-404, XP002466067, ISSN: 0143-3334, DOI: DOI:10.1093/CARCIN/BGI237 [retrieved on 2005-10-11]
- JOHANSSON P ET AL: "Confirmation of a BRAF mutation-associated gene expression signature in melanoma", PIGMENT CELL RESEARCH, MUNKSGAARD INTERNATIONAL PUBLISHERS, CAMBRIDGE, MA, DK, vol. 20, no. 3, 1 June 2007 (2007-06-01), pages 216-221, XP002527391, ISSN: 0893-5785, DOI: DOI:10.1111/J.1600-0749.2007.00375.X
- OTA T ET AL: "COMPLETE SEQUENCING AND CHARACTERIZATION OF 21,243 FULL-LENGTH HUMAN CDNAS", NATURE GENETICS, NATURE PUBLISHING GROUP, NEW YORK, US, vol. 36, no. 1, 1 January 2004 (2004-01-01), pages 40-45, XP008055982, ISSN: 1061-4036, DOI: DOI:10.1038/NG1285
- DE ROOCK WENDY ET AL: "Effects of KRAS, BRAF, NRAS, and PIK3CA mutations on the efficacy of cetuximab plus chemotherapy in chemotherapy-refractory metastatic colorectal cancer: a retrospective consortium analysis.", THE LANCET ONCOLOGY AUG 2010 LNKD- PUBMED:20619739, vol. 11, no. 8, August 2010 (2010-08), pages 753-762, XP002619537, ISSN: 1474-5488

## Description

### Field

The present invention relates to the field of cancer prognosis, diagnosis and treatment response. More particular, the invention relates to a method for typing an RNA sample of an individual suffering from cancer. The invention furthermore relates to a set of genes for use in typing an RNA sample of said individual. Said typing allows discrimination of individuals with and without an activated EGFR-pathway in likely-responders and non-responders towards anti-EGFR and/or EGFR pathway therapy.

The Epidermal Growth Factor Receptor (EGFR), also termed HER1 in humans, is a cell-surface receptor for epidermal growth factors (EGF-family) and plays a key role in cell migration, adhesion, and proliferation (Mendelsohn and Baselga, 2006. Semin Oncol 33: 369-385; Hynes and Lane, 2005. Nat Rev Cancer 5: 341-354). EGFR is a member of the ErbB subfamily of receptors, constituting four closely related receptor tyrosine kinases: EGFR (ErbB-1), HER2/c-neu (ErbB-2), Her 3 (ErbB-3) and Her 4 (ErbB-4). Clinical studies have shown that HER-2/Neu is over-expressed in up to one-third of patients with a variety of cancers, including B-cell acute lymphoblastic leukemia (BALL), breast cancer, ovarian cancer and lung cancer, and that these patients are frequently resistant to conventional chemo-therapies. The receptor tyrosine kinases work mainly through two pathways: 1.) by activating Ras GTPase and 2.) by activating phosphatidylinositol-3-OH kinase (PI(3)K). Each of these proteins then activates a number of downstream effectors. The identification of the ErbB receptor family as oncogenes has led to the development of many targeted anticancer therapeutics including gefitinib and erlotinib for lung cancer (Shepherd et al., 2005. N Engl J Med 353: 123-132), and lapatinib and Herceptin for breast cancer (Nahta et al., 2006. Nat Clin Pract Oncol 3: 269-280). Many therapeutic approaches are aimed directly at EGFR with Cetuximab (Erbitux) (Cunningham et al., 2004. N Engl J Med 351: 337-345), and Panitumumab (Van Cutsem et al., 2007. J Clin Oncol 25: 1658-1664) as examples of monoclonal antibody inhibitors in colon cancer. Several other antibodies have been developed and are successfully used for a variety of cancer (e.g.: Trastuzumab / Herceptin, Pertuzumab / Omnitarg / rhuMab-2C4, Cetuximab / Erbitux / IMC-C225, Panitumumab / Abenix / ABX-EGF, and also ZD6474). Additionally, small inhibitors of the tyrosine kinase domain of the EGFR have been developed, such as Lapatinib (Tykerb, GW572016), Gefitinib (Iressa, ZD1839), and Erlotinib (Tarceva, OSI-774). Several more anti-EGFR-antibodies and small molecule inhibitors are presently in clinical trials.

As many of these targeted therapies will be enormously cost-intensive (approximately 25.000 Euro per year per patient) it becomes more and more important to identify patients who will benefit from these drugs - or to eliminate patients who will certainly not profit from them. At the same time, novel compounds are being tested in the clinic that inhibit these pathways more downstream, i.e. at the level of phosphatidylinositol 3-kinase (PI3K), MAP-ERK Kinase (MEK), BRAF or mammalian Target of Rapamycin (mTOR), and diagnostic tests will be required to ask which patients are most likely to benefit from these therapies also. BRAF inhibitors like Plexxikon's PLX-4032 for metastatic melanoma, received lots of attention in the media because of the big success in treating melanoma patients. It was only a phase I trial, but 70% of patients responded to the compound (Brower V 2010 JNCI J Natl Cancer Inst 102 (4): 214-215). Key to the success of Plexxikon's drug seems to be its ability to target patients with BRAF mutation. The identification of patients who have a pathway activation through, for example, the BRAF mutation is therefore important.

IHC studies and gene amplification analysis indicate that over-expression of EGFR is not predictive for the response to these antibodies (Adams and Maughan, 2007. Expert Rev Anticancer Ther 7: 503-518; Chung et al., 2005. J Clin Oncol 23:1803-1810; Mitchell et al., 2007. J Clin Oncol 25:184s). On the other hand, several studies have shown that patients with KRAS mutation do not respond to Certuximab or Panitumumab (Benvenuti et al., 2007. Cancer Res 67: 2643-2648; Di Fiore et al., 2007. Br J Cancer 96: 1166-1169; Lievre et al., 2006. Cancer Res 66: 3992-3995; De Roock et al., 2008. Ann Oncol 19(3): 508-515; Khambata-Ford et al., 2007. J Clin Oncol 25: 3230-3237; Amado et al., 2008. JCO 28: 1628-1634). Said KRAS mutation may also influence a response to Bevacizumab (anti-VEGF mAb) (Ince et al., 2005. J Natl Cancer Inst 97: 981-989). First results have shown that patients with activating mutations in PI3-Kinase will also not respond to these antibodies (Sartore-Bianchi et al., Cancer Res. 2009. 69(5):1851-7). More recently, it was shown that mutations in PI3K confer resistance to Herceptin in breast cancer (Berns et al., 2008. Cancer Cell 12: 395 - 402).

In phase 1 and 2 studies, many of these agents have been shown to be well tolerated, achieve plasma concentrations within the predictive active range based on preclinical studies, inhibit the target receptor in translational research studies, and induce tumor responses in some patients. In phase 3 studies, however, combinations of these drugs with chemotherapy did often not result in improved outcomes. The most relevant question for future studies is how to identify patients with susceptible tumors for potential inclusion in further clinical trials.

Therefore, there is a requisite to have methods and means that allow a stratification of patients in likely-responders and non-responders towards anti-EGFR and/or EGFR-pathway therapy.

The present invention as defined by the appended claims provides a method for typing a RNA sample of an individual suffering from cancer, or suspected of suffering there from, the method comprising (a) providing an RNA sample that is prepared from a tissue sample from said individual, said tissue sample comprising colorectal cancer cells or suspected to comprise colorectal cancer cells;
(b). determining RNA levels for a set of at least five genes selected from TRIM7, ACSL6, AGR2, AHR, AIFM3, ANXA10, ARID3A, ASCL2, ASXL1, AXIN2, BX427767, BX647159, C10orf47, C10orf59, C13orf18, C20orf142, CEBPA, CEP68, CR616309, DID01, DNAH2, DUSP18, EPDR1, ETV5, GGT7, GPR143, HOXD1, KCNK5, LOC157860, LOC388610, MLH1, MOCS3, MORC4, PCDH21, PLAGL2, POFUT1, PRDX5, PTPRO, QPRT, RAB27B, TRIM7, RAB32, REG4, RNF43, SHROOM2, SHROOM4, SLC39A5, SLC7A11, SMCR7L, SNHG11, SOX8, SPINK1, TFF2, TNNC2, TNNT1, UNKL, VAV3, ZIC5 and ZSWIM3 listed in Table 2 in said RNA sample, and
(c) typing said RNA sample on the basis of the RNA levels determined for said set of genes.

A method of the invention as defined by the appended claims allows identifying individuals with activating mutations in the EGFR pathway who are likely not to respond to anti-EGFR therapy or treatment. The term anti-EGFR therapy includes any small molecule-mediated therapy that targets an EGFR receptor such as, for example lapatinib, erlotinib and gefitinib, antibody-mediated therapy that targets an EGFR receptor such as, for example, Trastuzumab (Herceptin), Cetuximab, and Panitumumab, and could further include therapy that is aimed at inactivating mediators that are downstream of the receptor.

A method of the invention allows identifying individuals with activating mutations in the EGFR pathway who are likely to respond to small molecules inhibiting downstream targets in the EGFR pathway. The term downstream targets includes any small molecule-mediated therapy that targets components at the level of, or downstream of, the molecules BRAF Examples of these small molecules are, e g., BRAF inhibitors like PLX4032 (Plexxikon and Roche/Genentech),

The EGFR receptor is structurally composed of 3 principal domains: an extracellular ligand-binding domain, a transmembrane domain, and an intracellular domain with intrinsic tyrosine kinase (TK) activity. Ligand binding induces dimerization of a receptor, resulting in autophosphorylation of the intracellular domains which creates docking sites on the receptor for signal transducing molecules. These signal transducing molecules, constituting the EGFR pathway, comprise mitogen activated protein kinase (MAPK) pathways comprising the proto-oncogenes Ras and Raf, promoting gene expression and cell proliferation; PLC, modulating intracellular Ca2+ levels; and phosphatidylinositol 3-kinase (PI3K), resulting in the localized production of PIP3 [phophatitidylinositol (3,4,5)-triphosphate], which modulates AKT and JAK/STAT-mediated pathways, including mTOR.

The present gene expression signatures were identified after identifying cancer cells with an activating mutation in KRAS, BRAF, and PI3K. More specifically, mutation analysis in KRAS was performed by sequencing the whole gene to detect the activating mutations in codons 12, 13 and 61. For PI3K, mutations were analyzed in two previously reported "hotspot" regions in exons 9 and 20, corresponding to the accessory (helical) and catalytic domains of PIK3CA, respectively. As used herein, symbols for the phosphatidylinositol 3-kinase genes, PI3K, PI3-K and PIK3CA, are used interchangeably. BRAF mutations were analyzed in exon 15 to detect activating mutations that alter a valine encoded by codon 600.

Using RNA isolated from a training set of colorectal samples with and without one or more of said activating mutations in KRAS, BRAF and/or PI3-K, genes were selected of which the RNA levels were significantly related to the presence or absence of one or more of said activating mutations. The genes listed in Table 1 were shown to be predictive for the presence or absence of at least one activating mutation in KRAS, either because their RNA expression level is upregulated in a tissue sample comprising an activating mutation, or because their expression level is downregulated in a tissue sample comprising an activating mutation. The genes listed in Table 2 were shown to be predictive for the presence or absence of at least one activating mutation in BRAF. The genes listed in Table 3 were shown to be predictive for the presence or absence of at least one activating mutation in PI3-K. Transcriptional profiling of the samples used in this study indicated that gene expression patterns derived from one type of oncogenic mutation, in KRAS, BRAF or PI3-K, can also characterize other types of oncogenic mutations of genes in the same pathway.. Therefore, the invention provides a 3-way classification model for typing an RNA sample of an individual suffering from cancer, or suspected of suffering there from, in which the three gene expression signatures for activating mutations in KRAS, BRAF and PI3-K are combined. Using this classification model, a tumor can be classified as mutation-like or non-mutation like. A tumor is classified as mutation-like if the gene expression profile of the tumor indicates the presence of one or more EGFR or EGFR pathway activating mutation in KRAS, BRAF, PI3-K and/or other genes in the EGFR pathway. A tumor is classified as non-mutation like if the gene expression profile of the tumor does not indicate the presence of one or more activating mutation in KRAS, BRAF, PI3-K and/or other genes in the EGFR pathway.

A preferred set of genes in a method of the disclosure comprises at least 2 of the genes listed in Table, 1, Table 2, and/or Table 3, more preferred at least 5 of the genes listed in Table 1, Table 2, and/or Table 3, more preferred at least 10 of the genes listed in Table 1, Table 2, and/or Table 3, more preferred a set of genes in a method of the invention comprises at least 20 of the genes listed in Table 1, Table 2, and/or Table 3, more preferred at least 30 of the genes listed in Table 1, Table 2, and/or Table 3, more preferred at least 40 of the genes listed in Table 1, Table 2, and/or Table 3, A most preferred set of genes comprises all genes listed in Table 1, Table 2, and/or Table 3.

There is significant overlap between the three gene signatures. For example, tumor samples with a high score for the PIK3CA mutation signature are not only enriched for PIK3CA mutations (area under ROC curve 0.76) but are also enriched for KRAS (AUC 0.66) and BRAF (AUC 0.87) mutations. This also holds true for the KRAS mutation signature (AUC KRAS 0.77, AUC BRAF 0.76, AUC PIK3CA 0.72) and for the BRAF mutation signature (AUC BRAF 0.94, AUC KRAS 0.60, AUC PIK3CA 0.65). In addition, the signatures share some genes: the PIK3CA mutation signature has 21 shared genes with the KRAS and 11 shared genes with the BRAF signatures while the BRAF and KRAS signatures share 6 genes. Combined, the three gene signatures comprises a unique set of 155 genes (Table 9).

Therefore, a further preferred set of genes comprises at least 2 of the genes listed in Table 9, more preferred at least 5 of the genes listed in Table 9, more preferred at least 10 of the genes listed in Table 9, more preferred at least 20 of the genes listed in Table 9, more preferred at least 30 of the genes listed in Table 9, more preferred at least 40 of the genes listed in Table 9, A most preferred set of genes comprises all genes listed in Table 9. The genes listed in Table 9 provide a combined signature model that is representative of the three individual signatures. Tumors displaying a gene expression pattern similar to any of the three mutation signatures are classified as 'mutation-like', while tumors with a gene expression pattern that is negatively associated with all three mutation signatures (and therefore show a KRAS/BRAF/PIK3CA wildtype profile) are classified as 'non-mutation-like'.The combined signature model correctly predicted the presence of a mutation in KRAS, BRAF and/or PIK3CA for 158 of the 175 known mutation carriers (sensitivity of 90.3%).

A method according to the disclosure allows separation of samples comprising an activating mutation in the EGFR pathway from samples with no mutation in the EGFR pathway with 90.3% sensitivity and 61.7% specificity. Thus, approximately 38.3% of the samples that are typed as having an activating mutation in the EGFR pathway according to a present method have no activating mutation in codons 12, 13 or 61 of KRAS, exons 9 and 20 of PIK3CA, or V600 of BRAF. Without being bound by theory, said samples are likely to comprise activating mutations in other parts of the genes, or in other genes, or have other means of activating the pathway. Activation of the EGFR- pathway can also occur by over-expression of positive regulators, by activation of activating molecules, e.g. kinases, or inactivation of repressors, e.g. phosphatases, by epigenetic changes or by loss of expression of inhibiting regulators. The method is able to identify samples with an activation of the pathway independent of the cause of activation. Of the samples that are classified as having no activating mutation, in the EGFR pathway a low percentage (between 11,8 and 9.7%) are likely false negative patients (with mutations). Samples from patients that are typed as having an activating mutation in the EGFR pathway are not likely to respond to anti-EGFR therapy (De Rock W et al Lancet Oncol. 2010 Aug;11(8):753-62). Vice versa, samples from patients that are typed as not having an activating mutation in the EGFR pathway are likely to respond to anti-EGFR therapy.

A classification model according to the disclosure is preferred over currently known gene signatures. Said classification model combines the feature that every tumor can be classified as likely or not likely to respond to anti-EGFR therapy with a high sensitivity and a high specificity. The method using the classification model identifies all samples with activating mutation in the EGFR pathway independent of the mutated gene. Currently known gene signatures which classify tumors as likely or not likely to respond to anti-EGFR and/or EGFR pathway therapy using activating mutations in more than one gene of the EGFR pathway do not show the combination of a high sensitivity (90.3%) and a clinical relevant specificity (61.4%) which is achieved by the present classification model. A clinical relevant specificity is advantageous because it indicated that a low number of tumors are typed as having one or more activating mutations in KRAS, PI3K and/or BRAF, but which do not contain such mutations. Currently observed response rates to EGFR-therapies are around 25-30%, indicating that the tumor in ∼70% of patients has developed mechanism to be resistant to EGFR-inhibition. The 70% of non-responders are in good agreement with the observed 61.4% of patients who are having a downstream pathway activation.

In a present method a more clinical relevant specificity may result in a lower number of patients receiving anti-EGFR and/or EGFR pathway therapy who will not benefit from said therapy.

A tissue sample preferably comprises cancer cells, or is suspected to comprise cancer cells, selected from bladder cancer cells, melanoma cells, breast cancer cells, colon cancer cells, leukemia cells, lymphoma cells, squamous cancer cells, rectal cancer cells, colorectal cancer cells, pancreatic cancer cells, endometrial cancer cells, prostate cancer cells, renal cancer cells, skin cancer cells, liver cancer cells, head and neck cancer cells, and lung cancer cells. It is preferred that said cancer cells comprise colorectal cancer cells, breast cancer cells, or lung cancer cells.

A tissue sample is a clinically relevant sample that comprises a cancer cell or an expression product such as a nucleic acid from a cancer cell. For example, said tissue sample comprises stool or a blood sample from an individual suffering from cancer, or suspected to suffer from cancer. In a preferred embodiment, a tissue sample is obtained directly from the individual, for example by removal of a biopsy. In a most preferred embodiment, said tissue sample is obtained from a tumor after removal of the tumor from a patient. Said sample is preferably obtained from the tumor within two hours after removal, more preferably within 1 hour after removal. Before a tissue sample is obtained from a removed tumor, said tumor is preferably cooled and stored at about 0 -8°C.

A tissue sample can be processed in numerous ways, as is known to a skilled person. For example, it can be freshly prepared from cells or tissues at the moment of harvesting, or it can be prepared from samples that are stored at - 70°C until processed for sample preparation. Alternatively, tissues, biopsies, stool or blood samples can be stored under conditions that preserve the quality of the nucleic acid, including the messenger RNA. Examples of these preservative conditions are fixation using e.g. formaline, RNase inhibitors such as RNAsin (Pharmingen) or RNasecure (Ambion), aquous solutions such as RNAlater (Assuragen; US06204375), Hepes-Glutamic acid buffer mediated Organic solvent Protection Effect (HOPE; DE10021390), and RCL2 (Alphelys; WO04083369), and non-aquous solutions such as Universal Molecular Fixative (Sakura Finetek USA Inc.; US7138226).

The RNA level of at least two of the genes listed in Tables 1-3 and/or Table 9 can be determined by any method known in the art. Methods to determine RNA levels of genes are known to a skilled person and include, but are not limited to, Northern blotting, quantitative PCR, and microarray analysis.

Northern blotting comprises the quantification of the nucleic acid expression product of a specific gene by hybridizing a labeled probe that specifically interacts with said nucleic acid expression product, after separation of nucleic acid expression products by gel electrophoreses. Quantification of the labeled probe that has interacted with said nucleic acid expression product serves as a measure for determining the level of expression. The determined level of expression can be normalized for differences in the total amounts of nucleic acid expression products between two separate samples by comparing the level of expression of a gene that is known not to differ in expression level between samples.

Quantitative Polymerase Chain Reaction (qPCR) provides an alternative method to quantify the level of expression of nucleic acids. qPCR can be performed by real-time PCR (rtPCR), in which the amount of product is monitored during the reaction, or by end-point measurements, in which the amount of a final product is determined. As is known to a skilled person, rtPCR can be performed by either the use of a nucleic acid intercalator, such as for example ethidium bromide or SYBR® Green I dye, which interacts which all generated double stranded products resulting in an increase in fluorescence during amplification, or by the use of labeled probes that react specifically with the generated double stranded product of the gene of interest. Alternative detection methods that can be used are provided by dendrimer signal amplification, hybridization signal amplification, and molecular beacons.

Different amplification methods, known to a skilled artisan, can be employed for qPCR, including but not limited to PCR, rolling circle amplification, nucleic acid sequence-based amplification, transcription mediated amplification, and linear RNA amplification.

For the simultaneous detection of multiple nucleic acid gene expression products, qPCR methods such as reverse transcriptase- multiplex ligation-dependent amplification (rtMLPA), which accurately quantifies up to 45 transcripts of interest in a one-tube assay (Eldering et al., Nucleic Acids Res 2003; 31: e153) can be employed.

Microarray-based analyses involve the use of selected biomolecules that are immobilized on a surface. A microarray usually comprises nucleic acid molecules, termed probes, which are able to hybridize to nucleic acid expression products. The probes are exposed to labeled sample nucleic acid, hybridized, and the abundance of nucleic acid expression products in the sample that are complementary to a probe is determined. The probes on a microarray may comprise DNA sequences, RNA sequences, or copolymer sequences of DNA and RNA. The probes may also comprise DNA and/or RNA analogues such as, for example, nucleotide analogues or peptide nucleic acid molecules (PNA), or combinations thereof. The sequences of the probes may be full or partial fragments of genomic DNA. The sequences may also be in vitro synthesized nucleotide sequences, such as synthetic oligonucleotide sequences.

It is preferred that said RNA levels are determined simultaneously. Simultaneous analyses can be performed, for example, by multiplex qPCR, RNA sequencing procedures, and microarray analysis. Microarray analyses allow the simultaneous determination of the nucleic acid levels of expression of a large number of genes, such as more than 50 genes, more than 100 genes, more than 1000 genes, or even more than 10.000 genes, allowing the use of a large number of gene expression data for normalization of the genes comprising the set of genes according to the disclosure

In a preferred embodiment, therefore, said RNA levels are determined by microarray analysis.

A probe is specific for a gene listed in Tables 1-3 and/or Table 9. A probe is specific when it comprises a continuous stretch of nucleotides that are completely complementary to a nucleotide sequence of a RNA product of said gene, or a cDNA product thereof. A probe can also be specific when it comprises a continuous stretch of nucleotides that are partially complementary to a nucleotide sequence of a RNA product of said gene, or a cDNA product thereof. Partially means that a maximum of 5% from the nucleotides in a continuous stretch of at least 20 nucleotides differs from the corresponding nucleotide sequence of a RNA product of said gene. The term complementary is known in the art and refers to a sequence that is related by base-pairing rules to the sequence that is to be detected. It is preferred that the sequence of the probe is carefully designed to minimize nonspecific hybridization to said probe. It is preferred that the probe is, or mimics, a single stranded nucleic acid molecule. The length of said complementary continuous stretch of nucleotides can vary between 15 bases and several kilo bases, and is preferably between 20 bases and 1 kilobase, more preferred between 40 and 100 bases, and most preferred about 60 nucleotides. A most preferred probe comprises a continuous stretch of 60 nucleotides that are identical to a nucleotide sequence of a RNA product of a gene, or a cDNA product thereof.

To determine the RNA level of at least two of the genes listed in Tables 1-3 and/or Table 9, the RNA sample is preferably labeled, either directly or indirectly, and contacted with probes on the array under conditions that favor duplex formation between a probe and a complementary molecule in the labeled RNA sample. The amount of label that remains associated with a probe after washing of the microarray can be determined and is used as a measure for the level of RNA of a nucleic acid molecule that is complementary to said probe.

The determined RNA levels for at least two genes listed in Tables 1-3 and/or Table 9 can be normalized to correct for systemic bias. Systemic bias results in variation by inter-array differences in overall performance, which can be due to for example inconsistencies in array fabrication, staining and scanning, and variation between labeled RNA samples, which can be due for example to variations in purity. Systemic bias can be introduced during the handling of the sample in a microarray experiment. To reduce systemic bias, the determined RNA levels are preferably corrected for background non-specific hybridization and normalized using, for example, Feature Extraction software (Agilent Technologies), RMA normalization, or proprietary data analysis code. Other methods that are or will be known to a person of ordinary skill in the art, such as a dye swap experiment (Martin-Magniette et al., Bioinformatics 21:1995-2000 (2005)) can also be applied to normalize differences introduced by dye bias. Normalization of the expression levels results in normalized expression values.

Conventional methods for normalization of array data include global analysis, which is based on the assumption that the majority of genetic markers on an array are not differentially expressed between samples [Yang et al., Nucl Acids Res 30: 15 (2002)]. Alternatively, the array may comprise specific probes that are used for normalization. These probes detect RNA products from stable genes including known "housekeeping genes" such as glyceraldehyde-3-phosphate dehydrogenase and 18S rRNA levels, of which the RNA level is thought to be constant in a given cell and independent from the developmental stage or prognosis of said cell.
In a preferred embodiment, genes are selected of which the RNA expression levels are largely constant between individual tissue samples comprising cancer cells from one individual, and between tissue samples comprising cancer cells from different individuals. It will be clear to a skilled artisan that the RNA levels of said set of normalization genes preferably allow normalization over the whole range of RNA levels.

Said normalization preferably comprises LOESS or Lowess (LOcally WEighted Scatterplot Smoothing) a locally weighted polynomal regression normalization method to correct for e.g. unequal quantities of starting RNA, differences in labeling or detection efficiencies between the fluorescent dyes used, and systematic biases in the measured expression levels as well as intensity-dependent bias and systemic technical differences.
Said normalization considers the measurements of intensities on the array, but preferably considers a the measurement of a reference samples that is analyzed on the same microarray as the studied samples and is used for calculation of normalized gene expression levels, which are typically quantified as (log-)ratios between the sample and the reference channel.
A reference sample is preferably an RNA sample isolated from a tissue of a healthy individual, or an RNA sample from a cancerous growth of an individual of which the activating mutations in the EGFR pathway have been determined. A preferred reference sample comprises an RNA sample from a relevant cell line or mixture of cell lines. The RNA from a cell line or cell line mixture can be produced in-house or obtained from a commercial source such as, for example, Stratagene Human Reference RNA. A further preferred reference sample comprises RNA isolated and pooled from normal adjacent tissue from cancer patients.

A more preferred reference sample comprises an RNA sample from an individual suffering from cancer and in which the presence or absence of activating mutations in the EGFR pathway has been determined. In an even more preferred embodiment, said reference sample is a pooled RNA sample that is isolated from tissue comprising cancer cells from multiple individuals suffering from cancer and which cancer cells either have no activating mutations in the EGFR pathway, or have at least one activating mutation in the EGFR pathway. It is preferred that said multiple samples are pooled from more than 10 individuals, more preferred more than 20 individuals, more preferred more than 30 individuals, more preferred more than 40 individuals, most preferred more than 50 individuals. A most preferred reference sample comprises a balanced pooled RNA sample that is isolated from tissue comprising cancer cells from multiple individuals suffering from cancer and having no activating mutations in the EGFR pathway, and from individuals suffering from cancer having at least one activating mutation in the EGFR pathway.

To differentiate cancer cells with activating mutations in the EGFR pathway, which are likely not to respond to anti-EGFR therapy, from cancer cells that have no activating mutations in the EGFR pathway, the normalized RNA levels of at least two of the genes listed in any one of Tables 1-3 are analyzed and a score or index is calculated that quantifies or qualifies a studied sample. Said RNA levels are preferably analyses as (log-)ratio values that have been determined against a reference sample.

Typing of a sample can be performed in various ways. In one method, a coefficient is determined that is a measure of a similarity or dissimilarity of a sample with a previously established gene pattern that is specific of a certain cell type, tissue, disease state or any other interesting biological or clinical interesting samples group. Herein we call such a specific gene expression pattern a "profile template". Typing of a sample can be based on its (dis)similarity to a single profile template or based on multiple profile templates. In the disclosure the profile templates are representative of samples that have no activating mutation, and/or of samples that harbor no known oncogenic mutation in the EGFR pathway. Said profile templates are herein also called as "gene signatures" or "gene profiles".

A number of different coefficients can be used for determining a correlation between the RNA expression level in an RNA sample from an individual and a profile template. Preferred methods are parametric methods which assume a normal distribution of the data. One of these methods is the Pearson product-moment correlation coefficient, which is obtained by dividing the covariance of the two variables by the product of their standard deviations. Preferred methods comprise cosine-angle, un-centered correlation and, more preferred, cosine correlation (Fan et al., Conf Proc IEEE Eng Med Biol Soc. 5:4810-3 (2005)).

Preferably, said correlation with profile template is used to produce an overall similarity score for the set of genes that are used. A similarity score is a measure of the average correlation of RNA levels of a set of genes in an RNA sample from an individual and a profile template. Said similarity score can, for example, be a numerical value between +1, indicative of a high correlation between the RNA expression level of the set of genes in a RNA sample of said individual and said profile template, and -1, which is indicative of an inverse correlation. Preferably, an arbitrary threshold is used to differentiate between samples having an activating mutation in the EGFR pathway and samples not having an activating mutation in the EGFR pathway. Said threshold is an arbitrary value that allows discriminating between RNA samples from patients without an activating mutation in the EGFR pathway and RNA samples from patients with an activating mutation in the EGFR pathway. Said similarity threshold value is set at a value at which an acceptable number of patients with an activating mutation in the EGFR pathway would score as false negatives, and an acceptable number of patients without an activating mutation in the EGFR pathway would score as false positives. A similarity score is preferably displayed or outputted to a user interface device, a computer readable storage medium, or a local or remote computer system. Said similarity scores is herein also referred to as "signature score", or "index".

In another aspect, the disclosure provides a method of classifying an individual suffering from cancer, comprising classifying said individual as having an activating mutation in the EGFR pathway or not having an activating mutation in the EGFR pathway by a method comprising (a) providing an RNA sample from a said individual that is prepared from a tissue sample from said individual, said tissue sample comprising cancer cells or suspected to comprise cancer cells; (b) determining a level of RNA for a set of genes comprising at least two of the genes listed in Tables 1-3 and/or Table 9 in said sample; (c) determining a similarity value between a level of expression from the set of genes in said individual and a level of expression from said profile template that is representative of samples not having an activating mutation in the EGFR pathway; and (d) classifying said individual as having an activating mutation in the EGFR pathway if said similarity value is below a first similarity threshold value, and classifying said individual as not having an activating mutation in the EGFR pathway if said similarity value exceeds said first similarity threshold value.

In another example, step (c) comprises determining a similarity value between a level of expression from the set of genes in said individual and a level of expression from said profile template that is representative of samples having at least one activating mutation in the EGFR pathway; and (d) classifying said individual as having an activating mutation in the EGFR pathway if said similarity value is above a first similarity threshold value, and classifying said individual as not having an activating mutation in the EGFR pathway if said similarity value is below said first similarity threshold value.
Alternatively, step (c) comprises determining a first similarity value between a level of expression from the set of genes in said individual and a level of expression from said profile template that is representative of samples not having an activating mutation in the EGFR pathway; (d) determining a second similarity value between a level of expression from the set of genes in said individual and a level of expression from said profile template that is representative of samples having an activating mutation in EGFR pathway; (e) determining a difference value between the second similarity value and the first similarity value; and (f) classifying said individual as having an activating mutation in the EGFR pathway if said difference value exceeds a first similarity threshold value, and classifying said individual as not having an activating mutation in the EGFR pathway if said similarity value does not exceed said first similarity threshold value.

Classifying a sample as having an activating mutation in the EGFR pathway may indicate that the cancer is not responsive to anti-EGFR treatment. Such cancers are likely to respond to inhibition of the activating mutation by, e.g., inhibiting the mutated molecules, such as, for example, PI3K, BRAF, AKT, and MEK. Classifying a sample as having no activating mutation in the EGFR pathway may indicate that the cancer is responsive to anti-EGFR treatment.

The disclosure provides for a method of treating a cancer patient, comprising determining whether the level of expression of at least two genes of Tables 1-3 and/or Table 9, in a tissue sample from said individual, said tissue sample comprising cancer cells or suspected to comprise cancer cells, correlates with the level of these genes in a profile template that is representative of samples having no activating mutation in the EGFR pathway; and administering anti-EGFR treatment if the level of expression of the two genes correlates with the level of expression in a profile template that is representative of samples. The term "correlates" is used to indicate that the determined similarity is above a pre-determined threshold. A skilled person will understand that said method comprises prescribing anti-EGFR treatment if the level of expression of the two genes correlates with the level of expression in a profile template that is representative of samples not having an activating mutation in the EGFR pathway.

It will be clear to a skilled person that the level of expression of at least two genes of Tables 1-3 and/or Table 9 can also be compared to the level of expression of these genes in different profile templates to determine a difference value, as explained hereinabove.

The combined EGFR pathway signature distinguishes patients with an activating mutation in the EGFR-pathway from patients not having an activating mutation in the EGFR pathway with a high specificity. The EGFR pathway can be inhibited at multiple key-points in the pathway. The best treatment option for patients is provided by inhibition of the EGF-receptor if no mutations in the EGFR pathway are present. These patients have no activating mutation in the EGRF pathway as disclosed by the signature.

Patients with activating mutations downstream of the receptor or other activating mechanism, as identified by the signature, may benefit from therapy that inhibits the pathway at the level of the activating mutation or downstream of the pathway. The disclosure thus further provides a method of treating a cancer patient as described above, wherein the level of expression of the at least two genes of Tables 1-3 and/or Table 9 from said sample does not correlate with the level of expression in a reference sample having no activating mutation in the EGFR pathway. In these patients, the presence of a mutation in a molecule in the EGFR signaling pathway is determined in a tissue sample, said tissue sample comprising cancer cells or suspected to comprise cancer cells. An inhibitor of said mutated molecule or of a molecule downstream of said mutated molecule is then administered. A number of activating mutations in the EGFR signaling pathway have been previously characterized, e.g., PI3K, BRAF, AKT, and MEK (see, e.g., Gandhi et al. 2009 PLoS ONE 4(2): e4576) and methods to detect such mutations are well-known in the art. Preferably, the inhibitor targets the mutated protein or a protein immediately downstream in the EGFR signaling pathway. Inhibitors of the EGFR pathway are known and include, e.g., PLX-4032 for the inhibition of BRAF, BEZ235 for the inhibition of AKT, and CI-1040 for inhibition of MEK. Preferably, said inhibitors are small molecule inhibitors, but also include antisense nucleic acids and protein therapeutics, such as antibodies and protein display scaffold proteins that bind the mutated protein or a protein downstream and inhibit signaling.

Clinical data have shown that patients having a high level of epiregulin (EREG) and/or amphiregulin (AREG) can benefit from EGFR-antibodies. (Khambata-Ford et al. 2007. J Clin Oncol 25: 3230-3237). In data provided here, patients with a non-activated EGFR pathway as defined by the signatures have high expression of EREG and AREG. This indirectly supports the observation that the patients identified as having a non- activated EGFR-pathway are likely to respond to EGFR-inhibition. Examples of EGFR-antibodies are Cetuximab (Erbitux) and Panitunumab (Vectibix) that are FDA-approved and routinely used for the treatment of metastatic colorectal cancer. Other examples of monoclonals in clinical development are zalutumumab (proposed trade name HuMax-EGFr), nimotuzumab (BIOMAb EGFR) and matuzumab.

Another method of inhibition is using small molecules to inhibit the EGFR tyrosine kinase, which is on the cytoplasmic side of the receptor. Without kinase activity, EGFR is unable to activate itself, which is a prerequisite for binding of downstream adaptor proteins. Most advanced examples are Gefitinib (Iressa), erlotinib (Tarceva) and lapatinib (Tykerb) (mixed EGFR and ERBB2 inhibitor).

In a further preferred embodiment, a method further comprises determining the expression level of EREG, and AREG. Over-expression of any one of these markers, preferably all two markers, compared to the level of expression of that marker in a reference sample from a patient not having an activating mutation in the EGFR pathway, was found to be indicative for a likeliness to respond to anti-EGFR therapy.

In yet a further preferred embodiment, a method of classifying an individual suffering from cancer, comprising: classifying said individual as having an activating mutation in the EGFR pathway or not having an activating mutation in the EGFR pathway by a method comprising: (a) providing a sample from a said individual that is prepared from a tissue sample from said individual, said tissue sample comprising cancer cells or suspected to comprise cancer cells; (b) determining a level of expression of epiregulin (EREG) and/or amphiregulin (AREG) in said sample; (c) determining a similarity value between a level of expression from a set of genes comprising at least two genes of Tables 1-3 and/or Table 9 in said individual and a level of expression from said set of genes in a profile template representative of samples not having an activating mutation in the EGFR pathway; and (d) classifying said individual as having an activating mutation in the EGFR pathway if said similarity value is below a first similarity threshold value, and classifying said individual as not having an activating mutation in the EGFR pathway if said similarity value exceeds said first similarity threshold value.

It is preferred that the level of expression of epiregulin (EREG) and amphiregulin (AREG), is determined in said sample. Said level of expression can be determined at the protein level, or preferably at the RNA level.

As is indicated in the results, the methods clearly differ from other methods for differentiating between colorectal cancer patients. For example, the methods allow differentiation of patients with high expression levels of EREG and AREG into a group that has a good response to cetuximab treatment versus a group that has a poor response to cetuximab treatment. Thus, although the high EREG and AREG patients in general can benefit from EGFR-targeting therapy such as anti-EGFR antibodies, the methods allow identifying high EREG and AREG patients that have a good response to treatment with EGFR-targeting therapy, versus high EREG and AREG patients that have a poor response to treatment with EGFR-targeting therapy.

Similarly, the methods allow differentiating of patients without KRAS mutations into a group that has a good response to cetuximab treatment versus a group that has a poor response to cetuximab treatment. Thus, although KRAS wild-type patients in general can benefit from EGFR-antibodies, the methods allow identifying KRAS wild-type patients that have a good response to treatment with EGFR-targeting therapy, versus KRAS wild-type patients that have a poor response to treatment with EGFR-targeting therapy.

### FIGURE LEGENDS

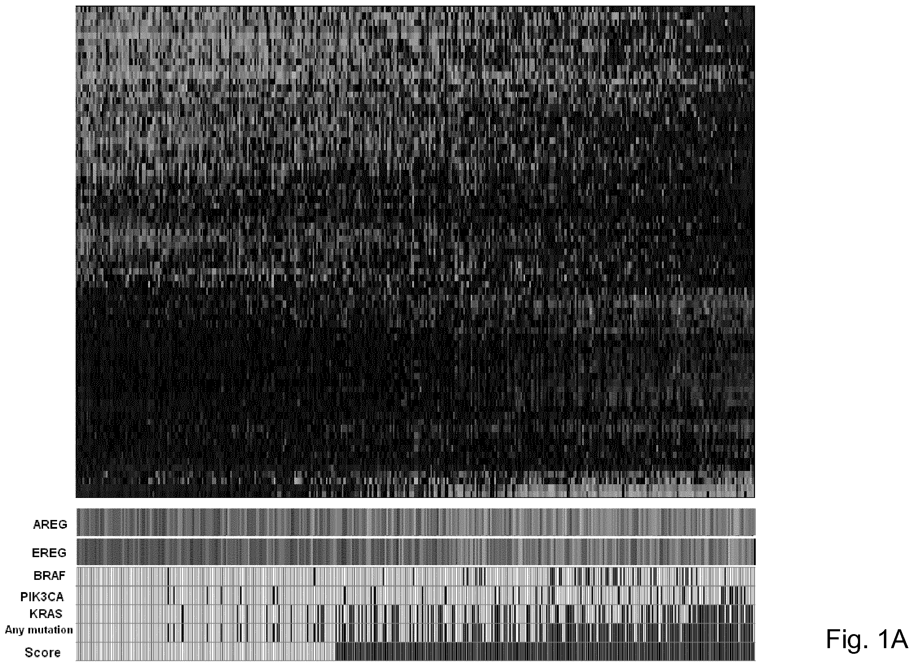

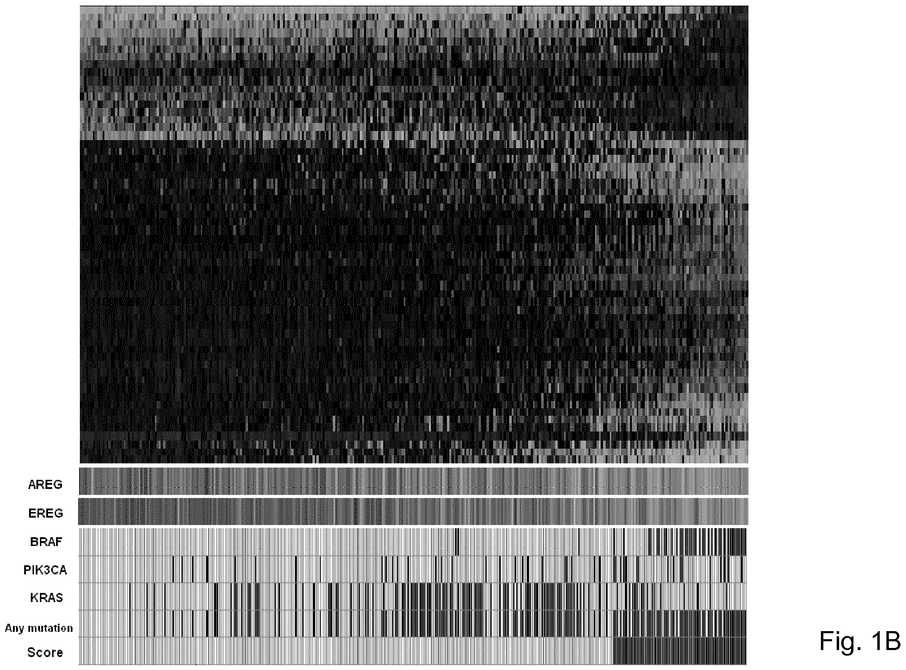

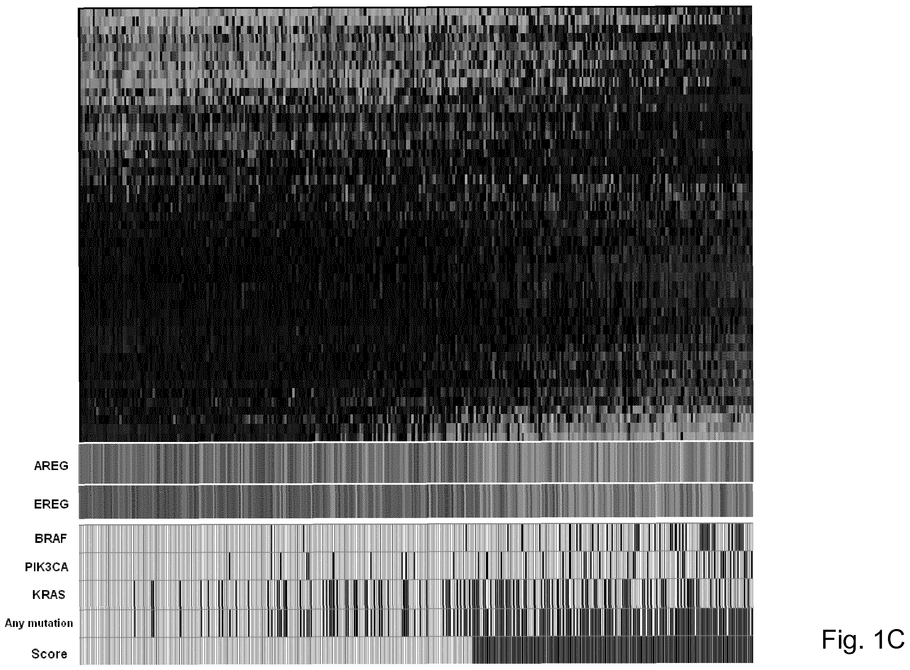

**Figure 1****.** KRAS, BRAF and PIK3CA activating mutation(-like) gene expression signatures.
   Gene expression signatures specific for tumors that harbor activating mutations in (A) KRAS (75-gene KRAS signature), (B) BRAF (58-gene BRAF signature) or (C) PIK3CA (49-gene PIK3CA signature). The heatmap represents relative gene expression levels of the signature genes across 381 colon tumor samples. Tumors are sorted according to the signature outcome (score). High gene expression is colored in red, low expression in green. *Score:* tumor classified as mutation-like by the signature score are displayed as a black box, tumors that are classified as non-mutation-like are displayed as white boxes. *KRAS, BRAF* and *PIK3CA:* Presence of oncogenic mutations as measured by sequence analysis are indicted by corresponding black boxes. *Any mutation*: tumors carrying any of KRAS/BRAF/PIK3CA mutation are indicated by black boxes. *AREG, EREG*: Expression levels of AREG and EREG,

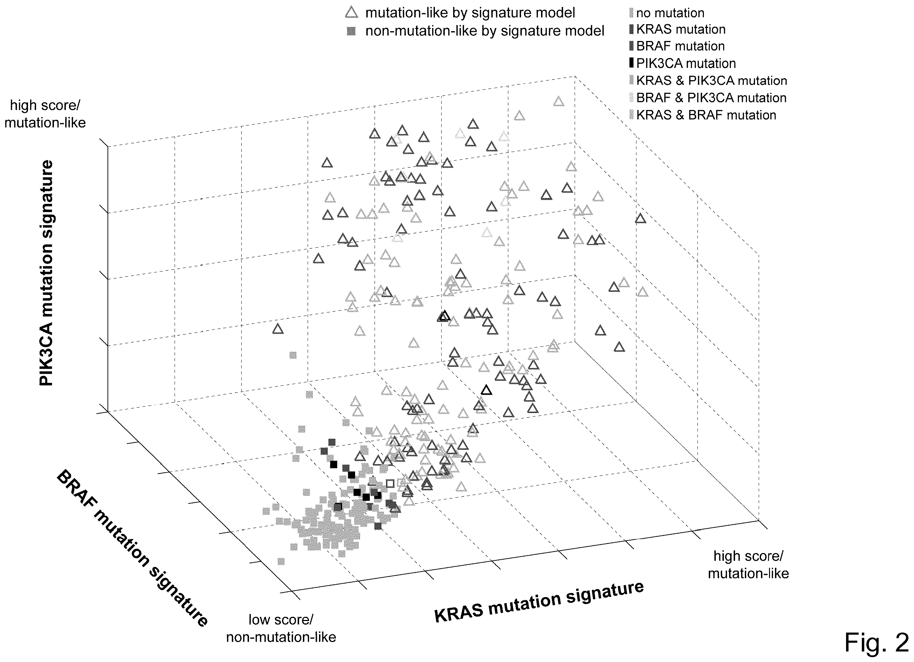

**Figure 2****.** An integrative classification model for KRAS, BRAF and PIK3CA oncogenic mutation-like tumor samples.
   Classification model for classification of 381 colon tumor samples based within a so called '3D oncogenic space' based on its association with the developed gene expression mutation(-like) signatures for *KRAS* (x-axis), *BRAF* (y-axis) and *PIK3CA* (z-axis). Each point represents a single tumor sample and is colored according to its mutation status as measured by sequence analysis. The shape of the point represents that classification outcome based on the developed model in which triangles indicate tumors that are classified as mutation-like for the EGFR pathway by either *KRAS, BRAF* and/or *PIK3CA;* squares indicate tumors that are classified as non-mutation-like by all of the three signatures.

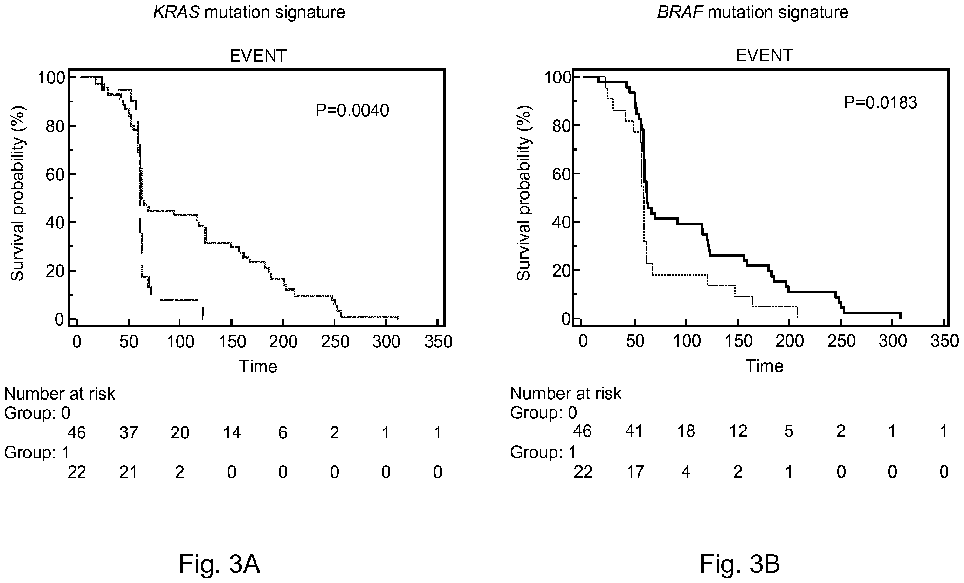

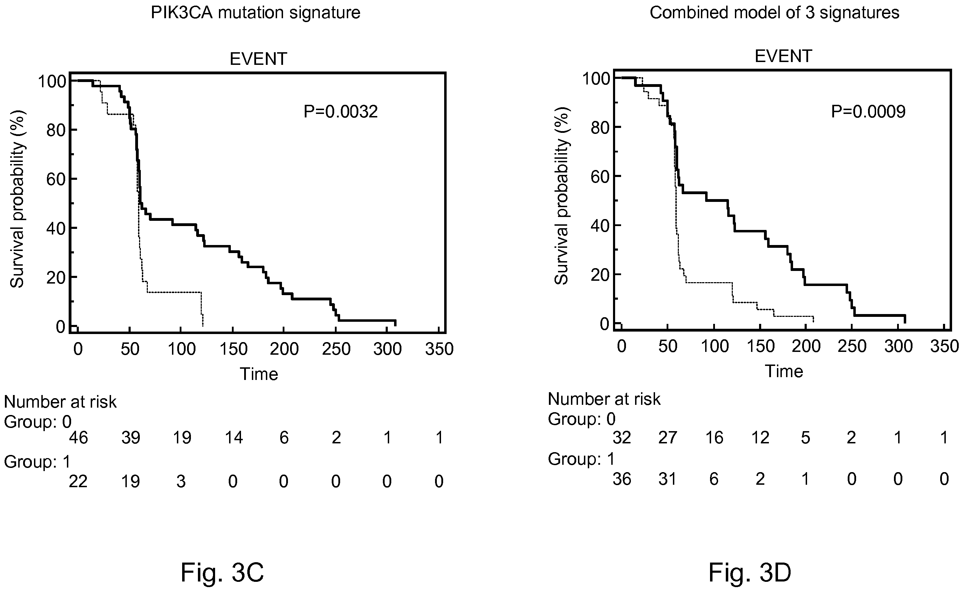

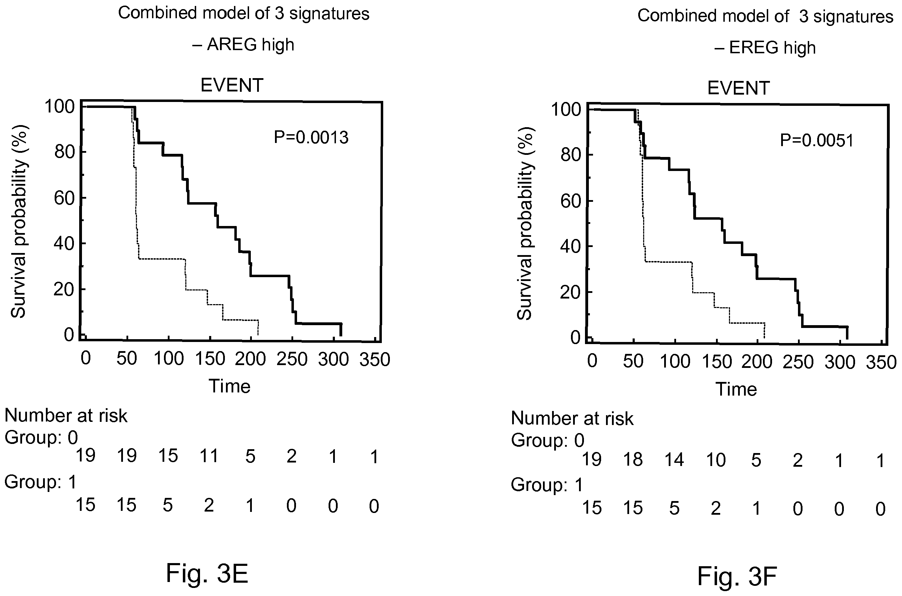

**Figure 3****.** Classification by KRAS, BRAF and PIK3CA mutation signatures is associated with response to cetuximab treatment.
   Kaplan-Meier (KM) survival analysis of 68 metastatic colon cancer patients who have received cetuximab treatment. Tumor sample have been classified in *silico* as non-mutation-like (class 0, solid line) or as mutation-like (class 1, dashed line) by the (A) KRAS mutation signature, (B) BRAF mutation signature, (C) PIK3CA mutation signature, and by (D) the combined classification model. In addition, KM survival curves are shown for classification by the combined signature model of AREG-high (E) and EREG-high (F) samples groups.
**Figure 4****.** Mean performance of random combination (100) of genes selected from within the **(A)** *KRAS* **(B)** *BRAF* and **(C)** *PIK3CA* mutation signatures. The simulation was performed for 2-20 genes. Blue line indicates sensitivity, green line indicates specificity, and red line indicates overall performance. X-axis represent gene set size (2 to 20), Y-axis shows the signature performance.
**Figure 5****.** Log-rank statistics of predicted cetuximab treatment response across thousand random combinations of KRAS 2 genes/BRAF 2 genes/PIK3CA 2 genes.
**Figure 6****.** KM survival curves for classification by the combined signature model of the KRAS wild-type sample group. Tumor samples were classified *in silico* as non-mutation-like (group 0, solid line) or as mutation-like (group 1, dashed line).

### EXAMPLE 1

### Patients and Material and Methods

### Patients

A total of 381 fresh frozen tumor samples from patients with colorectal cancer were collected. Most patients had stage II (n=209) and stage III (n=114) colorectal cancer; 50 patients had stage I and 8 patients stage IV cancer. All tissue samples were collected from patients with appropriate informed consent. The study was carried out in accordance with the ethical standards of the Helsinki Declaration and was approved by the Medical Ethical Board of the participating medical centers and hospitals.

### Mutational analysis

Mutation analysis was performed on all samples using a sequencing approach. Only mutations that have been proven and published to constitutively activate the signal transduction pathway were included.

Mutation analysis in KRAS was performed by sequencing the whole gene to detect the activating mutations in codon 12 and 13 (most common) and 61. The primers used were aggcctgctgaaaatgaxtg (left primer) and tggtgaatatcttcaaatgatttagt-M13 (right primer). The product size was 297 bp. For PI3-Kinase, mutations were analyzed in two previously reported "hotspot" regions in exons 9 and 20, corresponding to the accessory (helical) and catalytic domains of PIK3CA, respectively. For Exon 9, the used primers were ccacgcaggactgagtaaca (left primer) and ggccaatcttttacccaagca-M13 (right primer). For the Exon 20, the left primer used was (tgagcaagaggctttggagt) and the right primer was (agtgtggaatccagagtgagc-M13). BRAF mutations were analyzed in exon 15 after amplification of cDNA to detect a V600E activating mutation. Primers used were (primer 1) 5'-tgatcaaacttatagatattgcacga and (primer 2) 5'- tcatacagaacaattccaaatgc. Amplified products were purified using a Macherey-Nagel NucleoFast® purification kit and checked on gel for size and yield. Approximately 16-20ng of each product was used in a reverse sequence reaction using the M13 primers. The Mutation Surveyor Software was used for Genotyping analysis.

### Microarray hybridization

Several cryo-sections were cut from the frozen tissue. The first and the last slice of 5µM were used for H&E staining and to assess the tumor cell percentage. A macro-dissection (30 slices of 30µ) was used for RNA isolation and the percentage of tumor cells in the sample was determined by a trained pathologist in the H&E stained sections. RNA was isolated following standard protocols and tested for RNA quality using a BioAnalyzer (Agilent). Two-hundred nanogram total RNA was amplified using the QuickAmp Labeling Kit (Agilent Technologies). Cyanine 3-CTP or Cyanine 5-CTP (GE Health Care) was directly incorporated into the cRNA during in vitro transcription. A total of 825 ng of Cyanine-labeled RNA was co-hybridized with a standard reference to Agilent high-density 4x44k oligo nucleotide microarrays at 65 degrees Celsius for 17 hrs and subsequently washed according to the Agilent standard hybridization protocol (Agilent Oligo Microarray Kit, Agilent Technologies). All samples contained at least 40% tumor cells. The reference comprised of a pool of 47 colorectal cancer specimens that included samples with and without known oncogenic mutation in KRAS, BRAF and PI3K. The reference was processed and labeled in the same manner as the test samples.

### Microarray image analysis and data pre-processing

Fluorescence intensities on scanned images were quantified, values corrected for background non-specific hybridization, and normalized using Agilent Feature Extraction software (version 9.5) according to the manufactures recommended settings for the Agilent Whole Genome 4x44k microarray. The default normalisation procedure for this microarray includes a linear and a Lowess component, which corrects for any difference in Cy3/5 dye incorporation and centers the final profile at 0 (log-scale, Cy5/Cy3). This process is described in the Agilent Feature Extraction Reference Guide. Normalised gene expression ratios from each hybridisation were combined to produce a single gene expression profile, per patient, using Matlab software (MathWorks, Inc, Natick, MA). Normalized gene expression data (log-scaled) was used for development of EGFR pathway related gene profiles (also called gene signatures).

### Development of activating mutation(-like) gene signatures.

To identify gene signatures that characterize the gene expression pattern associated with KRAS, PIK3CA and BRAF oncogenic mutations, we used a 10-fold cross validation procedure that was repeated a thousand times to generate a robust gene selection. Within the training procedure, genes were ranked by the average p-values from a Student T-test between (1) tumors carrying the investigated type of mutations (n=115 KRAS mutants, n = 42 BRAF mutants, n= 44 PIK3CA mutants, Table 4) and (2) tumors carrying no known oncogenic mutation in KRAS, BRAF and PIK3CA mutation (n = 206). The optimal number of signature genes was selected to reach a maximal overall accuracy. The selected set of optimal gene probes was used for construction of a nearest centroid based classification method similar as in (Glas et al., 2006. BMC Genomics 7: 278) to score all tumor samples for their correlation with the KRAS activated mutation signature (75 genes), the BRAF activated mutation signature (58 genes) and the PI3CA activated mutation signature (49 genes). Samples were classified within the corresponding mutation-like group if their signature score exceeded a pre-defined optimized threshold.

### Signature validation on cetuximab response data

Publicly available Affymetrix gene expression and clinical data from 80 colon cancer patients treated with cetuximab (Khambata-Ford et al., 2007. J Clin Oncol 25: 3230-3237) were downloaded via the Gene Expression Omnibus (GEO) with accession number GSE5851. Clinical data regarding cetuximab treatment response was available for 68 patients. We could identify 49 of the 75 *KRAS* signature genes, 37 of the 59 BRAF signature genes and 34 of the 49 PIK3CA signature genes. Readout of the signature scores on the Affymetrix data by the nearest centroid classifier methods was done in a similar fashion as used for the in-house Agilent microarray data. Samples were ranked according to the signatures scores and classified as mutation-like (n=22) and non-mutation-like (n=46) based on the 33-percentile.

### Results & Discussion

### Mutation analysis

Oncogenic KRAS mutations were most frequently observed in our studied sample population with 115 tumors (30.2%) harboring an activating *KRAS* mutation (Table 4). Mutations in PIK3CA and BRAF were less frequent and occur in 44 (11.6%) and 42 (11%) samples. In total 175 (45.9%) of the studies tumors harbor at least one oncogenic mutation and 206 (54.1%) samples showed no known oncogenic mutation in any of the PIK3CA, KRAS or BRAF genes.

### Gene expression signatures of oncogenic mutations

KRAS, BRAF and PIK3CA mutation status as measured by sequence analysis were used to develop three signatures that characterize the gene expression patterns for each of these three types of oncogenic mutations and are able to classify the samples as non-mutation(-like) and activating mutation(-like) tumors. Using a 10-fold cross validation procedure, three sets of genes were identified that were optimally suited for constructing an activating mutation signature for KRAS (75 genes, Figure 1A, Table 1), an activating mutation signature for BRAF (58 genes, Figure 1B, Table 2) and an activating mutation signature for PIK3CA (49 genes, Figure 1C, Table 3). Performance of the three developed mutation signature was assessed by the respective samples used for development of the signature (Table 5). The KRAS mutation signature correctly classified 105 of the 115 tumor with an oncogenic KRAS mutation (sensitivity of 91.3%) and showed on overall accuracy of 72.3 percent. The BRAF mutation signature correctly classified 38 of the 42 mutated tumor samples (sensitivity 90.5%) and 189 of the 206 non-EGFR-mutated tumor (specificity 91.8%) resulting in an overall accuracy of 91.5% (Table 3). The PIK3CA mutation signature showed a sensitivity of 75% with an overall accuracy of 79.2%.

Compared to the KRAS signature and the PIK3CA signature, the BRAF mutation specific signatures has the best performance with an overall accuracy of 91.5% versus 72.3% for *KRAS* and 79.2% for *PIK3CA.* This indicates that gene expression patterns resulted from BRAF mutations are likely more explicit than those resulting from KRAS or PIK3CA mutations. Indeed the more explicit BRAF signature can be observed in Figure 1B with two large distinctive groups and a much smaller ambiguous region when compared with KRAS (Figure 1A) and PIK3CA profiling (Figure 1C). The biological reason behind this finding might be that KRAS and PIK3CA function more upstream in EGFR signaling transduction pathway, while the BRAF gene is positioned more downstream. As such, genes located on the upstream part as the EGFR pathway, such as KRAS and PIK3CA, may synergize a wide range of different signal transduction pathways and transcriptional effects, while genes located on the downstream region, such as BRAF, might be more committed to a limited number of signal transduction pathways, thereby resulting in a more explicit gene expression pattern.

Functional analysis of the mutation signatures indicated that the oncogenic mutations within KRAS, BRAF and PIK3CA result in rather broad functional effects (Table 6). The three signatures are only weakly enriched for functional categories that include general cellular processes such as cell cycle, cellular movement, lipid metabolism. This suggests that the gene expression changes caused by the oncogenic mutation in the EGFR pathway change a wide range of processes that may be relevant to tumorgenesis.

### An integrative 3-way classification model to identify EGFR oncogenic mutation-like tumors

The three individual mutation-like gene signatures for KRAS, BRAF and PIK3CA have been developed for an accurate classification of only one gene within the EGFR pathway. However, a close inspection of the tumor classification (Figure 1) indicates that gene expression pattern derived from only one type of oncogenic mutation can also characterize other types of oncogenic mutations on the same pathway. For example, tumor samples with a high score for the PIK3CA mutation signature (Figure 1C) are enriched for PIK3CA mutations (AUC = area under ROC curve 0.76, n=381) but are also enriched for KRAS (AUC 0.66) and BRAF (AUC 0.87) mutations. Similarly, this also holds true for the KRAS mutation signature (AUC for KRAS 0.77, AUC for BRAF 0.76, AUC for PIK3CA 0.72) and for the BRAF mutation signature (AUC for BRAF 0.94, AUC for KRAS 0.60, AUC for PIK3CA 0.65). This result demonstrates two fundamental concepts. First, oncogenic mutations downstream of EGFR such as KRAS/BRAF/PIK3CA converge at a transcriptional level and display a similar gene expression pattern. Secondly, a gene expression pattern extracted from one type of known oncogenic mutation on EGFR pathway can to some extent predict the presence of other types of oncogenic mutations on EGFR pathway. One can speculate that mutations may not be limited to oncogenic mutations within the KRAS, BRAF and PIK3CA genes, but could also include other genes on the EGFR pathway such as mTOR and AKT, or other type of genetic lesions resulting in an activated EGRF signature.

For a comprehensive identification of tumors that harbor activating mutation(s) in the EGFR signaling pathway, we have combined the three individual KRAS, BRAF and PIK3CA mutation signatures into an integrative 3-way classification model (Figure 2). In this combined model the gene expression pattern of each tumor is scored for its association with all of the three oncogenic mutation signatures and placed into a so-called '3D oncogenic space' (Figure 2). Consequently, if a tumor displays a gene expression pattern similar to any of the three mutation signatures it is classified as *'mutation-like',* while a samples that harbors a gene profile that is negatively associated with all three mutation signatures (and therefore shows a KRAS/BRAF/PIK3CA wildtype profile) is classified as *'non-mutation-like'.* The combined classification model correctly predicted the presence of a mutation in KRAS, BRAF and/or PIK3CA for 158 of the 175 known mutation carriers (sensitivity of 90.3%). Interestingly, 79 of the 206 tumors that showed no known oncogenic mutation by sequence analysis were classified as mutation-like by the gene signature model (specificity of 61.7%). We propose that this latter group of tumors samples might display an mutation-like gene signature due to unknown activating mutations in the EGFR pathway that are yet unknown and therefore not covered in the mutation sequence analysis.

### Association of the KRAS/BRAF/PIK3CA mutation signatures with response to anti-EGFR therapies

To learn whether classification of colon tumor samples based on the developed mutation signatures is associated with response to anti-EGFR therapies, we investigated the correlation of the signatures scores with expression levels of amphiregulin (AREG) and epiregulin (EREG), two known biomarkers that indicate response to antibody drugs targeting the EGFR-pathway (Khambata-Ford et al., 2007. J Clin Oncol 25: 3230-3237). Both response biomarkers showed a significant correlation with the scores of the three KRAS, BRAF and PIK3CA mutation signatures (AREG: Pearson correlations -0.39, -0.42 and - 0.47; EREG: -0.51, -0.53 and -0.58, respectively). Tumors with a low mutation signature score (thus predicted to have non-mutation based EGFR activation) showed a relative high expression of AREG/EREG (Figure 1) and are expected to benefit better from EGFR receptor targeted drugs (Jacobs B et al J Clin Oncol. 2009 Oct 20;27(30):5068-74).

We also validated the mutation signature classifications with response to cetuximab (Erbitux®) treatment in patients with metastatic colorectal cancer. For this purpose we used a publicly available data from 80 colon cancer patient who have been treated with cetuximab (Khambata-Ford et al., 2007. J Clin Oncol 25: 3230-3237). Tumor samples were analyzed *in silico* for the developed KRAS, BRAF and PIK3CA mutation signatures (see methods for details). Clinical response data of the cetuximab treatment was available for 68 of the 80 samples and was used to assess the predictive value of the mutation signatures. The 68 tumors were ranked based on their gene signature and classified as mutation-like and non-mutation-like based on the 33-percentile. Kaplan-Meier survival analysis resulted in a significant better survival of patients classified as non-mutation-like (n=46) compared to patients classified as mutation-like (n=22) by the gene signatures (KRAS signature, P=0.0040, Figure 3A; BRAF signature, P=0.0183, Figure 3B; PIK3CA, P=0.0032, Figure 3C). The combined 3-way classification model for identification of any mutation within the EGFR-pathway showed the strongest predictive value (P=0.0009, Figure 3D) and confirmed that the developed mutation signature is able to identify patients that benefit from anti-EGFR targeted drugs.

We further evaluated whether the prediction of cetuximab treatment by the combined signature model was independent of AREG and EREG expression levels. For this purpose, the 68 tumors were arbitrarily split into an AREG-high (n=34) and an AREG-low (n=34) group based on the median AREG expression level. Interestingly, within the AREG high-group, the mutation signature model remain significantly associated with survival of treated patients (P=0.0013, Figure 3E). This analysis was repeated for EREG expression levels and also within the EREG-high patient group the gene signature was able to identify patients who benefited from cetuximab treatment (P=0.0051, Figure 3F). Classification by the signature model showed no predictive value within the AREG-low (P=0.99) and EREG-low groups (P=0.35), probably because this group has little benefit from EGFR-inhibition.

These results indicate that, although classification by the gene signature model is associated with AREG/EREG expression, it likely holds additive predictive value to further stratify patients that benefit most from anti-EGFR targeted therapies.

### EXAMPLE 2

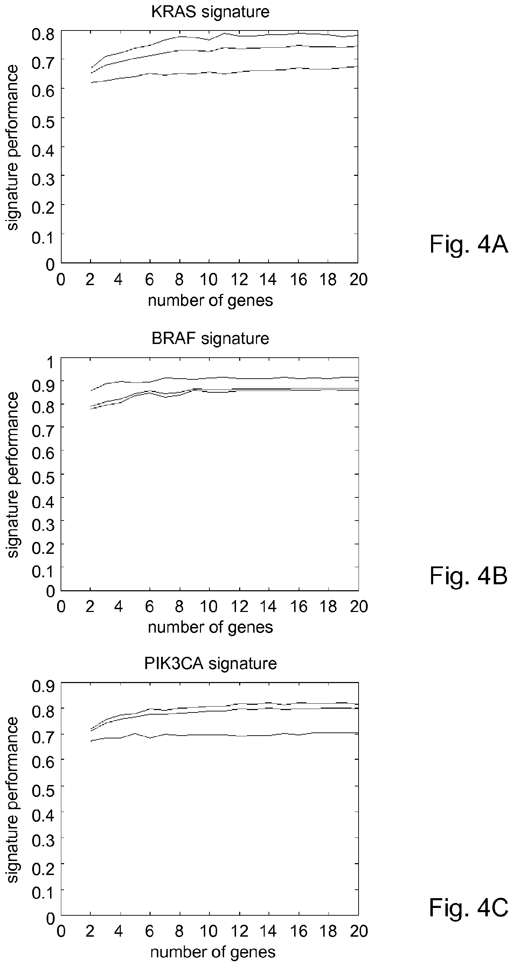

To determine the minimal number of signature genes that are required for construction of a EGFR related mutation signature, subsets of randomly selected genes from within the three signature genes sets (*KRAS*: 75 genes, *BRAF*: 58 genes, and *PIK3CA:* 49 genes) were analyzed for their performance to identify the samples' corresponding mutation status. To find the minimal number of genes that is needed to reach an overall performance above 60 percent, the mutation signature performance (measured as sensitivity, specificity and overall accuracy) was calculated based on: hundred random computer generated subsets from the respective KRAS, BRAF and PIK3CA signature genes consisting of 20 genes, hundred random sets of 19 genes, hundred sets of 18 sets, etc etc, down to the smallest gene set size of only two genes (as two genes are minimally required for construction of a gene signature). Figure 4 shows that the performance of the KRAS, BRAF and PIK3CA mutation signatures decreases only marginally in case of a lower number of genes is selected from within the signature gene sets, and remains well above 60 percent for overall accuracy. Importantly, a minimal KRAS, BRAF and PIK3CA signature consisting of only two random signature genes (Table 7) already shows a mean overall performance of 65, 78, and 71 percent, respectively.

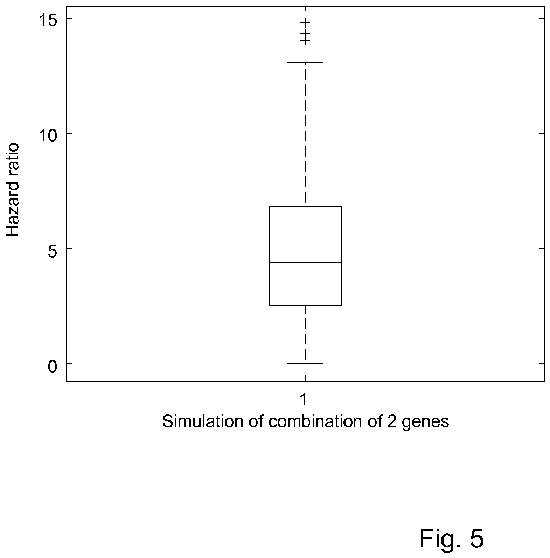

We further analyzed the clinical performance of a random subset of 2 signature genes for prediction of cetuximab treatment response. This analysis was done in a similar approach as has been applied for the complete signature sets (see above) and made use of the Kambata-Ford publicly available dataset. Survival analysis of 1000 computer generated subsets of signature genes that consisted of two random genes from the KRAS signature set, two random genes from the BRAF signature set and two random genes from the PIK3CA signature set indicated that for 90.9 percent of the thousand simulations the non-mutation-like signature group (predicted as responders) showed a better survival than the mutation-like group (predicted as non-responders). Calculated log-rank statistic of the thousand survival simulations are show in Figure 5 and shows a median log-rank statistic of 4.39 (Note: in this analysis a log-rank statistic greater than 1 is representative for a poorer survival in the mutation-like group and thus predicted as non-responders). These results indicate that, although the highest performance of the EGFR mutation signature genes is achieved used the complete set of 75 KRAS genes, 58 BRAF genes and/or 49 PIK3CA genes, the use of only 2 signature genes from each set already result in an classification that is predictive for the response to cetuximab.

### EXAMPLE 3

### Validation of the individual mutation signature and the combined EGFR oncogenic classification model on independent samples

The developed oncogenic mutation signatures for KRAS, BRAF and PIK3CA have been validated in an independent set of 80 samples of colorectal cancer patients. These samples have been analyzed for their EGFR pathway related gene expression similarly as the samples on which the signature were developed (see example 1) except that microarray hybridization was performed on the custom-made diagnostic 8-pack platform (Agendia) instead of on the Agilent 44K full genome array. Mutation status of KRAS, BRAF and PIK3CA was performed by sequence analysis, similarly as described above (example 1).

All three individual mutation signatures showed a significant association with the corresponding oncogenic mutation status in the independent validation samples, with an AUC of 0.83 for the KRAS signature, 0.95 for BRAF and 0.71 for PI3KCA (Table 8). Binary classification of the validation samples as mutation-like or non-mutation like based on the signature thresholds as have been developed on the training samples, resulted in a sensitivity and specificity of 93% and 53% for KRAS, 100% and 88% for BRAF, and 69% and 61% for PIK3CA classification (Table 8). Interestingly, validation of the predictive value of the three individual signatures for classification of any of KRAS, BRAF or PIK3CA mutation showed a similar performance. This result confirms the notion that a mutation-specific signature can also be used for classification of other mutation within the EGFR pathway. The combined 3-way classification model to identify samples with any of the three oncogenic genes showed a sensitivity of 93% thereby correctly classifying 37 of the 40 samples with an oncogenic mutation. The model's specificity of 60% indicates that a substantial subset of the samples (24 out of 40) that do not harbor a known oncogenic mutation by sequencing analysis do express mutation-like gene expression patterns. This might be caused by yet unknown oncogenic mutation in either KRAS, BRAF or PIK3CA, or other genes within the EGFR pathway or other means to (constitutively) activate the pathway.

### EXAMPLE 4

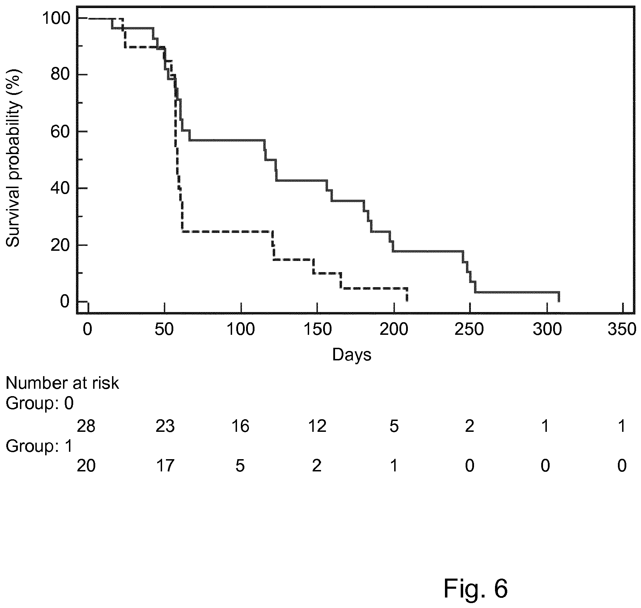

The publicly available data from 80 colon cancer patient who have been treated with cetuximab (Khambata-Ford et al., 2007. J Clin Oncol 25: 3230-3237) were additionally analysed to investigate whether the prediction of cetuximab treatment by the combined signature model was independent of mutation status of KRAS. The combined classification model showed a significant performance within KRAS wild-type patients (HR=2.49 p=0.009, Figure 6), indicating an association with survival beyond KRAS wild-type status. As expected, almost all samples with a KRAS mutation were classified as mutation-like by the combined signature (16 of 20) and within this group the gene signature model showed no association with treatment response (p=0.63). No patient in this dataset had a BRAF mutation and the status of PI3KCA mutation was not known for the dataset.

### TABLES

**Table 1 KRAS mutation gene signature**

| **ID** | **Name** | **Probe sequence** |
|---|---|---|
| AK022594 | AK022594 | CCTACTCAAGATGGCCACATTTCAATCCAGGCTCTTGAAGACAGGGTCTAATGAAAGGAT |
| AK130878 | AK130878 | GGAGGAGTTTGGATTGGAAACACCAGTCACAAGGACAGAATGAAGCCTGAATGAAAGTCT |
| NM_001164 | APBB1 | GAGAGATCTAATCCCTTCAAGGAAGTGATAACACTGGAGTGGTAACAAGAGGAGCAGGAA |
| NM_024590 | ARSJ | ACATGTGAACAGCTTGCACCTCATTTTACCATGCGTGAGGGAATGGCAAATAAGAATGTT |
| NM_015338 | ASXL1 | AATAGGGTTGTCTTTCCTATGAAAATGCCATCTGTAGACCTTGTGAGTCAGCCGTCCAGA |
| NM_001677 | ATP1B1 | AACTGTCATACGTATGGGACCTACACTTAATCTCTATGCTTTACACTAGCTTCTGCATTT |
| BC030102 | BC030102 | GCACTTGGATGGATTGGACCTATAGTTGTTATATATGGATATTGTCTAATTTATTTATTA |
| NM_030766 | BCL2L14 | AAGTGGTGATGTTGTTTCAAACGTTCAGAACAGATACCATCATCCTGCCTTTGTTAGCTG |
| NM_001202 | BMP4 | GGACACGAGACTGGTCCACCACAATGTGACACGGTGGGAAACTTTTGATGTGAGCCCTGC |
| BX647159 | BX647159 | AGGTTTTCTCTCTTGGTCCTACCTTGTGTATGTAGAAAGTTAATGTAAGCTTTTCTTCTG |
| NM_013399 | C16orf5 | GTGAATGATCAGACCCTGGTAGCTAAGAAGGAACTTGTCCCTTTGAGTCAGTGTGCAGAC |
| NM_016470 | C20orf111 | TACATGGAGTATTACTTGTATATTCCCAAGAAAATGTCCCACATGGCAGAAATGATGTAC |
| NM_001080472 | C20orf142 | TCTGGGTGTTGATGTTTCTGTGCACAGCTGTTTATTTCCACAACTTGTCCCAGAAGGTGT |
| NM_015511 | C20orf4 | TTCCTCTCTAGACAAGTACACAGGCCTGCCACCTGACATCAAACTGTTGTACTATGATCA |
| NM_001218 | CA12 | GTTCCCCGAGAACTTTCTGATTCACAGTCTCATTTTGACAGCATGAAATGTCCTCTTGAA |
| NM_000719 | CACNA1C | GAGTTTTACCAACCATTGTGTATGCCCAATAATTTGTTATCATTTCCTTAGGTAGTAACC |
| NM_032587 | CARD6 | ACTAGCTTCTGCTAATAGCCCCAATTTGCTTGAATGGGAAAACTCTCTCATTTGACCCTT |
| AK098629 | CNNM3 | AGACAGTGTGGTCTGTTGACAGTATGGAATATTCTTTACCCATCAGATATGATCTGTATG |
| NM_000784 | CYP27A1 | AGCTATTTGCTACATCCTGTTCGAGAAACGCATTGGCTGCCTGCAGCGATCCATCCCCGA |
| AL390175 | DKFZp547K054 | AAAGAATATCTGAGACGACGTTTGGGAGCAATGCTCTTTACCACGTTCACCAAGTCGTCT |
| NM_012242 | DKK1 | AATAACACCTTCCAAAAACCTGGAGTGTAAGAGCTTTGTTTCTTTATGGAACTCCCCTGT |
| NM_001946 | DUSP6 | GCTTGTGTTGTCGCAAAGGGGATAATCTGGGAAAGACACCAAATCATGGGCTCACTTTAA |
| NM_207032 | EDN3 | AAGTTATTACAGGTATAAAAGTGATGACCTATCATGAGGAAATGAAAGTGGCTGATTTGC |
| NM_006208 | ENPP1 | CCATCATAGGAATGGGCATTTGAACTTTGAAACTGAATGTGGTGATTACACTTCATGCTG |
| NM_021727 | FADS3 | AACTTTGAAGTGGAAAATCTGGCGTACATGCTGGTGTGCATGCAGTGGGCGGATTTGCTC |
| NM_015566 | FAM169A | AGAAAGGCTTACCTTCTGTCATCAAGTGATTGTATCATCCTGGATCGTCATTTCCAAGGA |
| NM_015101 | GLT25D2 | AGCATTTTAGACTAGGACTGTTCTACTGTGAAGAAAGTTCTGTCTCCTTTAGCCCGGTTT |
| ENST00000371081 | GNAS | TAATATCCCTAGATGATGGCTACAGTTTCTCATCTTCCCGGCTATGTCCTATCACAATTT |
| NM_001030004 | HNF4A | CATGAAGAAGGAAGCCGTCCAGAATGAGCGGGACCGGATCAGCACTCGAAGGTCAAGCTA |
| NM_002147 | HOXB5 | AAATGTCTTGTGTATTTCTCTGTGAATCCATGGGTCTGGCTAGAGGGCCCAAAGCTTGTA |
| NM_018952 | HOXB6 | ATTAATTTTCTATGTGTTGTGTTTGTAGTCTTGTCTTAGCTCTGGACGTGAAATACTTCG |
| NM_024016 | HOXB8 | GCTGTGGGCAATTGTTACAAGTGTTCTTAGGTTTACTGTGAAGAGAATGTATTCTGTATC |
| NM_033439 | IL33 | AACTTCATTTCTGTATCACCTGACCTCTGGATGCCAAAACGTTTATTCTGCTTTGTCTGT |
| NM_018689 | KIAA1199 | TGTTTCACAGTACAGGATCTGTACATAAAAGTTTCTTTCCTAAACCATTCACCAAGAGCC |
| NM_002776 | KLK10 | TGCCTACCTCTTAGACATGTTGTGAGGAGACTATGATATAACATGTGTATGTAAATCTTC |
| NM_144947 | KLK11 | TCCTGGACTACAGGAGATGCTGTCACTTAATAATCAACCTGGGGTTCGAAATCAGTGAGA |
| NM_019079 | L1TD1 | TTCTACCCAGAAGGATGGACAGCTAATAGCGTACTTGGGGATGAGGAGCAAGGAATATTA |
| NM_001001552 | LEMD1 | TCTACCTGACTGTGGAAAATAAGTCGCTGTTTGGTTAAGTAATTTAGGAGCAAAGCAATG |
| XM_001715202 | LOC100130910 | CAACTTTTAAACATTGGTTACCTTTTGGGAGAATAAAGGGAATGGGGGCGATGATCACAG |
| ENST00000398035 | LOC120376 | CACAGTTTACATGTCACTATTTCCAATTTTCTGATGCTAAGCATTCATATGAAGTCCTCA |
| NM_000239 | LYZ | GATTATGGGATATTTCAGATCAATAGCCGCTACTGGTGTAATGATGGCAAAACCCCAGGA |
| NM_000260 | MYO7A | ATACCAACTGGGCCTCTGATGTTCTTCCAGTGAGGCATCTCTCTGGGATGCAGAACTTCC |
| THC2669157 | OIT3 | CAGCAAATGTACTAATTTACACTGTCACTGTATACTTCTCCCTATAACCTTGTAAGTGTT |
| NM_007350 | PHLDA1 | TACAGGATCTAAAAGGGTTTTCTTAGAAAGGGCAATATTGTCCAATGAAGTAAGCAGAAG |
| NM_080476 | PIGU | CGGACCCCTATGGAAATGCGTTACATCCCTTTGAAAGTGGCCCTGTTCTATCTCTTAAAT |
| NM_032562 | PLA2G12B | CATCTGTGCAGAGGAGGAGAAGGAAGAGTTATGAGGAAGAAGTGATTCCTTCCTGGTTTT |
| NM_002657 | PLAGL2 | AGAGAAAAGTACAAGACAGAAATCTTCTAGCACTTTGTAAACACAGTGAATAACCTCTTG |
| NM_172236 | POFUT1 | GGATCAGTTTCATGTGAGTTTCAACAAGTCGGAGCTTTTTACAGGCATTTCCTTCAGTGC |
| NM_080685 | PTPN13 | GCAGATCTTAGGGATGATTAAAGGCAGCATTTGATGATAGCAGACATTGTTACAAGGACA |
| NM_007238 | PXMP4 | CCCAAAGGATACTGCCTTCTCAAGATCATAGGCCTCAGACTCCAACTGGTGTTATCCCAG |
| NM_014899 | RHOBTB3 | ACACATGTATAAAGATGTTCACAGAGAAAGATGCTCTGTAGAGAATTTGCTACCGAAGTT |
| NM_198232 | RNASE 1 | GTTAGGGCTCCTATTCAACACACACATGCTTCCCTTTCCTGAGTCCCATCCCTGCGTGAT |
| NM_153225 | RPESP | TCCTCTCATTCAGCAGGTGGCCCGTGAGACACAGAATACATGTCTGTTTGCTAAAGTAAA |
| NM_005980 | S100P | CCAAAAGTGTTTGTTGGCAATTATTCCCCTAGGCTGAGCCTGCTCATGTACCTCTGATTA |
| NM_002639 | SERPINB5 | CATCAGGCACAACAAAACTCGAAACATCATTTTCTTTGGCAAATTCTGTTCTCCTTAAGT |
| NM_003059 | SLC22A4 | AAACAAGAGACTCAATGGAGACAGAAGAAAATCCCAAGGTTCTAATAACTGCATTCTGAA |
| NM_000112 | SLC26A2 | TCCTCCTTTGAAGCTAATGGCATTTGTATATACACACTGCAGCAGAGCTTGTAGCTGGAC |
| NM_021095 | SLC5A6 | TTTTTCTCGTCGCTTGCCAATCTGTTTTTTAAAGGATCAGGCTCGTAGGGAGCAGGATCA |
| NM_014331 | SLC7A11 | CATTTTGCTTTCCTAACCATTCAGTCAGGAATTAAAATATGGCATTGTATAACAACTGGG |
| NM_019844 | SLCO1B3 | TCCTGGTCTTTCACTGACAATTCCAACATTCTTTACTTACAGTGGACCAATGGATAAGTC |
| NM_032229 | SLITRK6 | TCCATACCTTGTAAATAAGTGCCTTACGTGAGTGTGTCATCAATCAGAACCTAAGCACAG |
| NM_003098 | SNTA1 | TCCTGCGACAGCCCTTCGAGAAGCTGCAGATGTCTTCAGATGACGGTGCCAGTCTCCTTT |
| NM_001042633 | SNX21 | CCATGATGAAGCCAAATCTGAACCCATGTCCTCACTTCAAGGTTATCAATCTTGGATTGT |
| NM_001034954 | SORBS1 | TTGGTACTTCAAGAAGGACAAAGCAGTTTGGTACTTTTCCAGGCAACTATGTAAAACCTT |
| NM_145263 | SPATA18 | ACTTGCTGTTTCTTCAGTTTCTAATTTGAGCTGTAACTACACAAGGAAAGCTAAATAGTC |
| NM_006804 | STARD3 | AACTGGAAGTTCGAGAAGAATAATGAATATGGGGACACCGTGTACACCATTGAAGTTCCT |
| THC2624002 | THC2624002 | CCAGACTGATGAAATTCCTTCCAGTTTCAGAAAAGATTATCACCAATCCCTACATTATAA |
| THC2634713 | THC2634713 | GAACCCAGTGATGGAGCCTTAAGTAAAGAGTATAATTTTGGTGTGTGGTTGGTAGAATCA |
| THC2699065 | THC2699065 | GGTTTATTGACATGGAATGATTTGACCAAGGTGATATAGTGGTTAAGCAGTTAAGTGATT |
| THC2708669 | THC2708669 | GAGGGACTATAAAGGCCAAGACCTTGTTCTTGCCATTTTAGAGATTCAGAATATAATCTA |
| NM_138554 | TLR4 | TTACTGAGTGTTTCAGAGTGTGTTTGGTTTGAGCAGGTCTAGGGTGATTGAACATCCCTG |
| NM_144649 | TMEM71 | AACCACGTTCTAAACCAAGTTTTTAATCTTCTTGGGGCTCGTAATTACCTTTCACTTTAA |
| BE893137 | TNIK | AGCAAGATATCCTCCTCATGGTCCCTTTAGCTCTCAAAAGCAATGAAATCCTCCTGTTCT |
| NM_032865 | TNS4 | GGGCTGGCAGAGGCCCCCATATACTCTGCTACAGCTCCTATCATGAAAAATAAAATGTTT |
| NM_021202 | TP53INP2 | GGGGGAAGAGTTTAAGTTATAGGGCATTTGGCTCAAATTTTAAAAGGCCTTTTGTTTACC |

**Table 2 BRAF mutation gene signature**

| **ID** | **Name** | **Probe sequence** |
|---|---|---|
| NM_001009185 | ACSL6 | AAACAAATAGAAGAGCTTTACTCAATCTCCATGTGAAGTTCAAGGAAAGTTCTTCTCAGT |
| NM_006408 | AGR2 | CTCCTCAATCTGGTTTATGAAACAACTGACAAACACCTTTCTCCTGATGGCCAGTATGTC |
| NM_001621 | AHR | AATGGCTTCGGACAAAATATCTCTGAGTTCTGTGTATTTTCAGTCAAAACTTTAAACCTG |
| NM_001018060 | AIFM3 | CTGGAGGAGCTGAAGTTTGTGGCTTTTTACACTAAAGGCGACGAGGTGATCGCCGTGGCC |
| NM_007193 | ANXA10 | TGGTGATGCTGAGGACTACTAAAATGAAGAGGACTTGGAGTACTGTGCACTCCTCTTTCT |
| NM_005224 | ARID3A | CAGCTGCCCATGAGCATTCGGATCAACAGCCAAGCCTCCGAAAGCCGCCAGGACTCTGCT |
| NM_005170 | ASCL2 | CTGCTGGAGGGACACTGCTGGCAAACGGAGACCTATTTTTGTACAAAGAACCCTTGACCT |
| NM_015338 | ASXL1 | AATAGGGTTGTCTTTCCTATGAAAATGCCATCTGTAGACCTTGTGAGTCAGCCGTCCAGA |
| NM_004655 | AXIN2 | TTTTGTCTTAAAGGTCTTGAGGGTTGACCATGTTGCGTCATCATCAACATTTTGGGGGTT |
| BX427767 | BX427767 | CAACTTGAATTTGATCCCATAAAGTCAGGCATCAGGAAGCCATTCAGAATTTTTCACCCT |
| BX647159 | BX647159 | AGGTTTTCTCTCTTGGTCCTACCTTGTGTATGTAGAAAGTTAATGTAAGCTTTTCTTCTG |
| NM_153256 | C10orf47 | AAGATCTCAGGGTAGGGAAAAACCAGTGAATGAGGTTACACAGCAGGAAAAGAACCGGTT |
| NM_018363 | C10orf59 | CCATTTGGAGTTACATACTTGGAACACAGCATTGAGGATGTGCAAGAGTTAGTCTTCCAG |
| NM_025113 | C13orf18 | TGTGTCAAGGAAAGGGCTTTATTTGTGAATTTTGCCAGAATACGACTGTCATCTTCCCAT |
| BC029662 | C20orf142 | TTGTATTTAGAGAATGGCTCTTGCCACTGTTATGTACACACAGATCGTGTGTGTAAACAA |
| NM_004364 | CEBPA | GCCTTGTTTGTACTGTATGCCTTCAGCATTGCCTAGGAACACGAAGCACGATCAGTCCAT |
| NM_015147 | CEP68 | TTACTTTCTTGGCTAACCAGTTTCTTAGAAGAAAATGTGTCAGGGACTTGGGGATCTACA |
| CR616309 | CR616309 | GCCCCATTTCAAGTATAACCAGGAGGGAAAATGGTGCTTGAAATAAGCATGCCACAAAGG |
| NM_033081 | DIDO1 | ATATGATCTTAAGAGTCTAAACATTCAAGAGACGAGGGCAAGAAAGCCAGTCACATGTAG |
| XM_085578 | DNAH2 | GCCGGACCTCACTCAGACTTTGACCTTGGCCGAATTTGTGTGATGTGGCCCTGGAGATAC |
| NM_152511 | DUSP18 | CTCCTTTCTGTGCACAGCACTTTATTGTTACAAAGTACTCTTCCAAAAAGTTACCCTGTG |
| NM_017549 | EPDR1 | TCTAGATGCTTCTACTGTTATGTTTTATCTGCCCATTTATCTTTCTTAGTTACCAGGAGA |
| NM_004454 | ETV5 | TTTATAGCTTTCTTAACCTACACATAGTCTATACATAGATGCATATTTTACCCCCAGCTG |
| NM_178026 | GGT7 | TGGCCCCGAGCTTAGGGATGTGCTTGCAAACCCTTCTCAAGGGTCTCACAACCCCAACAT |
| NM_000273 | GPR143 | ATATTCCTCAGACTCAACAATTCTTGTTCTTTAGAACTGTGTTCTCACCTTCCCAACACT |
| NM_024501 | HOXD1 | TTTTTTCTTTAAAAAAGCGGTTTCTACCTCTCTATGTGCCTGAGTGATGATACAATCGCT |
| THC2657554 | KCNK5 | CTGTCTCCAGGTAGGTGGACCAGAGAACTTGAGCGAAGCTCAAGCCTTCTCAACTCAAGG |
| AK025743 | LOC157860 | GGGGTTTAGGGTCGAGCTGTTCCTGATGTTTATCGGAGACTGGGATCAAAGCTATCCAGG |
| NM_001013642 | LOC388610 | GGTGGGGGACACACCTTTTTCTCAGGAAGAGGTGATGGCAATGTAAAACATCTAAGCAAA |
| NM_000249 | MLH1 | TGTGGGATGTGTTCTTCTTTCTCTGTATTCCGATACAAAGTGTTGTATCAAAGTGTGATA |
| NM_014484 | MOCS3 | ATTTGCAAACTGGGAAATGACTCACAGAAAGCCGTGAAGATCCTCCAGTCCTTATCAGCA |
| NM_024657 | MORC4 | CACCACACCAGAAATGGGACCTAAATTAAGGCTTAAAGAATGAGCATGGCCACACTGTCA |
| NM_033100 | PCDH21 | TTCAGCAGGTGGATCCATTCTTCGACCCCCAGATGTGACTCTAAAGAAGGCTGAAAATTT |
| NM_002657 | PLAGL2 | AGAGAAAAGTACAAGACAGAAATCTTCTAGCACTTTGTAAACACAGTGAATAACCTCTTG |
| NM_015352 | POFUT1 | ACTGGGAATACCGCAGTGAATAAGATAAACTCCGTGTCCTTGTAGAGCCTTCATTTTAGT |
| NM_012094 | PRDX5 | ATCTTTGGGAATCGACGTCTCAAGAGGTTCTCCATGGTGGTACAGGATGGCATAGTGAAG |
| NM_030667 | PTPRO | AACTGTTCTAGTTCTTGATGGTTTTCTTCCTTATTAACAGTTGGTGTTTCTTCCTTGGCC |
| NM_014298 | QPRT | GCCCTTGATTTCTCCCTCAAGCTGTTTGCCAAAGAGGTGGCTCCAGTGCCCAAAATCCAC |
| NM_004163 | RAB27B | ATGTATCTGCTAGACTCTACATAGAAACTGAACATCAAGAACCCCACCAAAATATTACTT |
| NM_006834 | RAB32 | ACCAAAGCTTTCCTAATGAAGAAAACGATGTGGACAAAATTAAGCTAGATCAAGAGACCT |
| NM_032044 | REG4 | CTCTTCCTTTCTGCTAGCCTGGCTAAATCTGCTCATTATTTCAGAGGGGAAACCTAGCAA |
| NM_017763 | RNF43 | GGCAGAATTACAGCTGAGCGGGGACAACAAAGAGTTCTTCTCTGGGAAAAGTTTTGTCTT |
| NM_001649 | SHROOM2 | GTGGTCATTTTGATGATATGTGTGTAAAATGTGAATAATCCAATTGGTGTCTGTACTCAG |
| NM_020717 | SHROOM4 | TGCCAGGTTTAACCACTCATAGCAACAAGACTTTTACCCAGAGACCAAAACCTATAGACC |
| NM_173596 | SLC39A5 | TCACGTGGATGGTCCTCCTGGGAGATGGTCTACACAACCTCACTGATGGGCTGGCCATAG |
| NM_014331 | SLC7A11 | CATTTTGCTTTCCTAACCATTCAGTCAGGAATTAAAATATGGCATTGTATAACAACTGGG |
| NM_019008 | SMCR7L | CAAGGATTTCTTATGGTGGTTTCAGTTTCATTTGCATAAAGGTATTGAGAGGGAACAAAA |
| NR_003239 | SNHG11 | CCTGGAGGGGCCTCCTCATGATTGTTTGGGGGTTGATCACAGACCAAGAGTGACGAGTGA |
| NM_014587 | SOX8 | CCTGCTGCCTTCACTCCTTTCTGACCAAGCAACGCTAACTTTTGTACAGATCGATTTGAT |
| NM_003122 | SPINK1 | GGAAACGCCAGACTTCTATCCTCATTCAAAAATCTGGGCCTTGCTGAGAACCAAGGTTTT |
| NM_005423 | TFF2 | TCCAACTTCATCTTTGAAGTGCCCTGGTGCTTCTTCCCGAAGTCTGTGGAAGACTGCCAT |
| NM_003279 | TNNC2 | GACTTCGACGAGTTCCTGAAGATGATGGAGGACGTGCAGTAAGGAGTGGACAGTCGCCTC |
| BC107798 | TNNT1 | TCCGAGCGTAAGAAGCCTCTGGACATTGACTACATGGGGGAGGAACAGCTCCGGGAGAAA |
| NM_203297 | TRIM7 | AGCTCTGCCTTGAGAGGTTGTGGGACCTCTGGATCCAGCTGACCTGACAGGTCATCTACT |
| NM_023076 | UNKL | AGCAGAGGGGATCCAACGTCAGAGCTTTTAGAATTACTTTTTTAAGCAGCTGTCTTCTGG |
| NM_006113 | VAV3 | TTGCCAACCCTGGTATGCTGGAGCAATGGAAAGATTGCAAGCAGAGACCGAACTTATTAA |
| NM_033132 | ZIC5 | GTCTTCAGATTGTTGTGATGAATATGAAACATGCAGACCCTCCTTTATAAAGAAAAAGAC |
| NM_080752 | ZSWIM3 | TGTGAGAGTTTAAAGTGGGCAGGACATACTAGGGTTTAGCATTTTAGCCAATGTCTTCCT |

**Table 3 PIK3CA mutation gene signature**

| **ID** | **Name** | **Probe sequence** |
|---|---|---|
| NM_015338 | ASXL1 | AATAGGGTTGTCTTTCCTATGAAAATGCCATCTGTAGACCTTGTGAGTCAGCCGTCCAGA |
| NM_001677 | ATP1B1 | AACTGTCATACGTATGGGACCTACACTTAATCTCTATGCTTTACACTAGCTTCTGCATTT |
| THC2708669 | AQP5 | GAGGGACTATAAAGGCCAAGACCTTGTTCTTGCCATTTTAGAGATTCAGAATATAATCTA |
| NM_001677 | ATP1B1 | AATAATTGCCAGGAGTACAGTGCTCTTGTTGATCTTGTATTCAGTCAGGTTAAAACAACG |
| NM_016470 | C20orf111 | TACATGGAGTATTACTTGTATATTCCCAAGAAAATGTCCCACATGGCAGAAATGATGTAC |
| NM_001080472 | C20orf142 | TCTGGGTGTTGATGTTTCTGTGCACAGCTGTTTATTTCCACAACTTGTCCCAGAAGGTGT |
| NM_015511 | C20orf4 | TTCCTCTCTAGACAAGTACACAGGCCTGCCACCTGACATCAAACTGTTGTACTATGATCA |
| NM_080748 | C20orf52 | ACTACATCTGTCCCTTCCCATCAATCCCAGCCCATGTACTAATAAAAGAAAGTCTTTGAG |
| NM_004929 | CALB1 | CTGATTCTATGCCTGTATTTCCAACAAACTTACTGTCAGAGAATATGACCTAAATCCATT |
| NM_030877 | CTNNBL1 | CAGGAAGATCAAGAAAGTGGGAACCACTGAGAAGGAACATGAAGAGCATGTCTGTTCGAT |
| NM_000784 | CYP27A1 | AGCTATTTGCTACATCCTGTTCGAGAAACGCATTGGCTGCCTGCAGCGATCCATCCCCGA |
| NM_033081 | DIDO1 | ATATGATCTTAAGAGTCTAAACATTCAAGAGACGAGGGCAAGAAAGCCAGTCACATGTAG |
| NM_052951 | DNTTIP1 | ATCTGAAGCTAGAGGAATTGAAATCCTTTGTCCTACCCTCCTGGATGGTGGAGAAGATGA |
| NM_001946 | DUSP6 | GCTTGTGTTGTCGCAAAGGGGATAATCTGGGAAAGACACCAAATCATGGGCTCACTTTAA |
| NM_014183 | DYNLRB1 | ACACTGAAGCGACTGCAGAGCCAGAAGGGAGTGCAGGGAATCATCGTCGTGAACACAGAA |
| NM_002212 | EIF6 | TCACCTTCCAAGTTGTTCCATGGGCTCCTGGCTCTGGACTGTGGCCAACCTTCTCCACAT |
| CR936791 | FAM169A | AGAAAGGCTTACCTTCTGTCATCAAGTGATTGTATCATCCTGGATCGTCATTTCCAAGGA |
| NM_001452 | FOXF2 | TGTGGGATCTTCGTTGCCTTCAGTAATCAGGGTGTGAAAAAAGCAGACAAGTTGTGTGTG |
| NM_001098722 | GNG4 | CAAACTCCATCCAGTACATTCTTTCTTCTTTCATGAAAGAGCTTGAGTTGGATGTAAATA |
| NM_000273 | GPR143 | ATATTCCTCAGACTCAACAATTCTTGTTCTTTAGAACTGTGTTCTCACCTTCCCAACACT |
| NM_014278 | HSPA4L | AGCTTTCCGGATAATTTTATATATCAAACATACAGGATGGATACATAGTTGGCAACAGTC |
| NM_021258 | IL22RA1 | ACCTACCTCATGGAGATTGTGGTGAAGATGAAATGAAGTCATGTCTTTAAAGTGCTTAAT |
| NM_003740 | KCNK5 | CTGTGAAATGTTTTAATGAACCATGTTGTTGCTGGTTGTCCTGGCATCGCGCACACTGTA |
| NM_198991 | KCTD1 | TTGATGGTACAGAGCCCATTGTTTTGGACAGTCTCAAACAGCACTATTTCATTGACAGAG |
| NM_018689 | KIAA1199 | AACGCTCCTCTGAAATGCTTGTCTTTTTTCTGTTGCCGAAATAGCTGGTCCTTTTTCGGG |
| NM_002776 | KLK10 | GCTGTCTACACCCAGATCTGCAAATACATGTCCTGGATCAATAAAGTCATACGCTCCAAC |
| NM_144947 | KLK11 | TCCTGGACTACAGGAGATGCTGTCACTTAATAATCAACCTGGGGTTCGAAATCAGTGAGA |
| THC2688670 | LOC646951 | CCTGGGTTGTTGTGCATATTAGGACGTAGTATGTGTGTTGAAACGCCCCATACAGAATGA |
| THC2669157 | OIT3 | CAGCAAATGTACTAATTTACACTGTCACTGTATACTTCTCCCTATAACCTTGTAAGTGTT |
| NM_032562 | PLA2G12B | CATCTGTGCAGAGGAGGAGAAGGAAGAGTTATGAGGAAGAAGTGATTCCTTCCTGGTTTT |
| NM_002657 | PLAGL2 | AGAGAAAAGTACAAGACAGAAATCTTCTAGCACTTTGTAAACACAGTGAATAACCTCTTG |
| NM_025225 | PNPLA3 | CTTGACTACTAAAAACGTCTCCATGGCGGGGGTAACAAGATGATAATCTACTTAATTTTA |
| NM_015352 | POFUT1 | TTCACTATTGTTATTTGAAAAAATATTTTTATTTGTAAGAGTTTTTCTTTATTTAAAATG |
| NM_004163 | RAB27B | ATGTATCTGCTAGACTCTACATAGAAACTGAACATCAAGAACCCCACCAAAATATTACTT |
| NM_198232 | RNASE1 | GTTAGGGCTCCTATTCAACACACACATGCTTCCCTTTCCTGAGTCCCATCCCTGCGTGAT |
| NM_153225 | RPESP | TCCTCTCATTCAGCAGGTGGCCCGTGAGACACAGAATACATGTCTGTTTGCTAAAGTAAA |
| NM_002639 | SERPINB5 | CATCAGGCACAACAAAACTCGAAACATCATTTTCTTTGGCAAATTCTGTTCTCCTTAAGT |
| NM_019844 | SLCO1B3 | TCCTGGTCTTTCACTGACAATTCCAACATTCTTTACTTACAGTGGACCAATGGATAAGTC |
| NR_003239 | SNHG11 | CCTGGAGGGGCCTCCTCATGATTGTTTGGGGGTTGATCACAGACCAAGAGTGACGAGTGA |
| NM_001034954 | SORBS1 | ACGTCGCCTTCCTATGTATGACGAAACAAGAAACAGAGATTTCCAATTGCTCTTTTGTCT |
| NM_145263 | SPATA18 | ACTTGCTGTTTCTTCAGTTTCTAATTTGAGCTGTAACTACACAAGGAAAGCTAAATAGTC |
| NM_001062 | TCN1 | CCAGAAAATGAATGATACTATATTTGGTTTCACAATGGAGGAGCGCTCATGGGGGCCCTA |
| NM_022748 | TNS3 | GCTGGTATAGGAAGTTTCTTTCTTCCTTTTGTGTTACATGTGCATTAAACAGAACAAGCT |
| NM_033342 | TRIM7 | CGTAACATACCAGTTAGGGCCTGCGGAAGCATCTTGTAATGGAACACATTACTATTTCTG |
| NM_130783 | TSPAN18 | TGCTCTGGCAACCCCATAAATGACACCTGAGCTCCGTAGAAGCTAAGCTCCTGAGACCCA |
| Unknown | Unknown | GCACCAGGGGGAGGATTTTCATCAAGCACCCACACCTCTTTAAGTTTGCAGCAGATCCTC |
| NM_021197 | WFDC1 | AAGCAGCTAGGTTGCAAGAACATTCCTCTACTTTCTGCTAAGCCTTGGAAACAGTTGGGA |
| NM_013313 | YPEL1 | GGAGTAATGTGCGAACTTTCCCTTCTCCTTCGAATGCTGTTTTGTGAAAGAAACTGTGAA |
| NM_080752 | ZSWIM3 | TGTGAGAGTTTAAAGTGGGCAGGACATACTAGGGTTTAGCATTTTAGCCAATGTCTTCCT |

**Table 4. Overview of oncogenic mutations**

| **Gene** | **Number of tumors harboring this mutation type (percentage of n=381 tumors)** |
|---|---|
| **KRAS** | 115 (30.2%) |
| **BRAF** | 42 (11.0%) |
| **PIK3CA** (exon 20 or exon 9) | 44 (11.6%) |
| **Any** known oncogenic mutation in PIK3CA, KRAS and BRAF. | 175 (45.9%) |
| **No** known oncogenic mutation in any of PI3KCA, KRAS and BRAF | 206 (54.1%) |

**Table 5. Performance of KRAS, BRAF and PIK3CA mutation signatures**

| | **Tumors with mutation** | **Tumors without any Kras/Braf/PIK3CA mutations** | **Number of genes** | **Sensitivity (one type of mutation)** | **Specificity** | **Accuracy** |
|---|---|---|---|---|---|---|
| **KRAS** | 115 | 206 | 75 | 91.3% | 61.7% | 72.3% |
| **BRAF** | 42 | 206 | 58 | 90.5% | 91.8% | 91.5% |
| **PIK3CA (exon 20 or exon 9)** | 44 | 206 | 49 | 75.0% | 80.1% | 79.2% |

**Table 6. Enriched molecular and cellular functions of the KRAS, BRAF and PIK3CA mutation signatures.**

| **KRAS signature** | | |
|---|---|---|
| **Name** | **p-value** | **Number of genes** |
| Cellular Movement | 1.04E-05 - 4.47E-02 | 8 |
| Cellular Development | 6.23E-05 - 4.78E-02 | 15 |
| Gene Expression | 4.61E-04 - 3.84E-02 | 6 |
| Cell Morphology | 7.94E-04 - 4.47E-02 | 8 |
| Lipid Metabolism | 1.07E-03 - 4.78E-02 | 10 |
| | | |

| **PIK3CA signature** | | |
|---|---|---|
| **Name** | **p-value** | **Number of genes** |
| Cell Cycle | 2.40E-03 - 1.67E-02 | 2 |
| Cell Death | 2.40E-03 - 4.69E-02 | 4 |
| Cell Morphology | 2.40E-03 - 3.07E-02 | 2 |
| Cell-To-Cell Signaling and Interaction | 2.40E-03 - 4.92E-02 | 3 |
| Cellular Assembly and Organization | 2.40E-03 - 3.77E-02 | 2 |
| | | |

| **BRAF signature** | | |
|---|---|---|
| **Name** | **p-value** | **Number of genes** |
| Cell Cycle | 8.03E-05 - 4.83E-02 | 10 |
| Gene Expression | 4.37E-04 - 4.78E-02 | 5 |
| Cellular Development | 2.47E-03 - 4.51E-02 | 9 |
| Cellular Growth and Proliferation | 2.47E-03 - 4.23E-02 | 13 |
| Carbohydrate Metabolism | 2.88E-03 - 1.15E-02 | 2 |

**Table 7. Mean performance of random combinations (100) of 2 genes selected from within the KRAS/BRAF/PIK3CA signatures.**

| | Sensitivity | Specificity |
|---|---|---|
| KRAS 2 genes for *KRAS* mutation | 61.9% | 66.8% |
| BRAF 2 genes for *BRAF* mutation | 85.4% | 77.5% |
| PIK3CA 2 genes for *PIK3CA* mutation | 67.1% | 71.8% |
| Combination of KRAS 2 genes/BRAF 2 genes/PIK3CA 2 genes for any oncogenic mutation | 78% | 56.6% |

**Table 8. Validation performance of EGFR mutation signatures on independent samples.**

| | **AUC** | **AUC for all 3 mutations** | **sensitivity** | **specificity** | **sensitivity for all 3 mutations** | **specificity for all 3 mutations** |
|---|---|---|---|---|---|---|
| **KRAS 75-gene** | 0.83 | 0.86 | 93% | 53% | | |
| **BRAF 58-gene** | 0.95 | 0.82 | 100% | 88% | | |
| **PIK3CA 49-gene** | 0.71 | 0.83 | 69% | 61% | | |
| **Combined signature model** | | | | | 93% | 60% |

| | | | | | | |
|---|---|---|---|---|---|---|
| Note: Sensitivity and specificity were determined by applying the signature threshold as has been developed on the training cohorts | | | | | | |

**Table 9. All genes included in the combined signature model**

| **ID** | **Name** | **Probe sequence** |
|---|---|---|
| NM_001009185 | ACSL6 | AAACAAATAGAAGAGCTTTACTCAATCTCCATGTGAAGTTCAAGGAAAGTTCTTCTCAGT |
| NM_006408 | AGR2 | CTCCTCAATCTGGTTTATGAAACAACTGACAAACACCTTTCTCCTGATGGCCAGTATGTC |
| NM_001621 | AHR | AATGGCTTCGGACAAAATATCTCTGAGTTCTGTGTATTTTCAGTCAAAACTTTAAACCTG |
| NM_001018060 | AIFM3 | CTGGAGGAGCTGAAGTTTGTGGCTTTTTACACTAAAGGCGACGAGGTGATCGCCGTGGCC |
| AK022594 | AK022594 | CCTACTCAAGATGGCCACATTTCAATCCAGGCTCTTGAAGACAGGGTCTAATGAAAGGAT |
| AK130878 | AK130878 | GGAGGAGTTTGGATTGGAAACACCAGTCACAAGGACAGAATGAAGCCTGAATGAAAGTCT |
| NM_007193 | ANXA10 | TGGTGATGCTGAGGACTACTAAAATGAAGAGGACTTGGAGTACTGTGCACTCCTCTTTCT |
| NM_001164 | APBB1 | GAGAGATCTAATCCCTTCAAGGAAGTGATAACACTGGAGTGGTAACAAGAGGAGCAGGAA |
| NM_005224 | ARID3A | CAGCTGCCCATGAGCATTCGGATCAACAGCCAAGCCTCCGAAAGCCGCCAGGACTCTGCT |
| NM_024590 | ARSJ | ACATGTGAACAGCTTGCACCTCATTTTACCATGCGTGAGGGAATGGCAAATAAGAATGTT |
| NM_005170 | ASCL2 | CTGCTGGAGGGACACTGCTGGCAAACGGAGACCTATTTTTGTACAAAGAACCCTTGACCT |
| NM_015338 | ASXL1 | AATAGGGTTGTCTTTCCTATGAAAATGCCATCTGTAGACCTTGTGAGTCAGCCGTCCAGA |
| NM_001677 | ATP1B1 | AACTGTCATACGTATGGGACCTACACTTAATCTCTATGCTTTACACTAGCTTCTGCATTT |
| NM_001677 | ATP1B1 | AATAATTGCCAGGAGTACAGTGCTCTTGTTGATCTTGTATTCAGTCAGGTTAAAACAACG |
| NM_004655 | AXIN2 | TTTTGTCTTAAAGGTCTTGAGGGTTGACCATGTTGCGTCATCATCAACATTTTGGGGGTT |
| BC030102 | BC030102 | GCACTTGGATGGATTGGACCTATAGTTGTTATATATGGATATTGTCTAATTTATTTATTA |
| NM_030766 | BCL2L14 | AAGTGGTGATGTTGTTTCAAACGTTCAGAACAGATACCATCATCCTGCCTTTGTTAGCTG |
| NM_001202 | BMP4 | GGACACGAGACTGGTCCACCACAATGTGACACGGTGGGAAACTTTTGATGTGAGCCCTGC |
| BX427767 | BX427767 | CAACTTGAATTTGATCCCATAAAGTCAGGCATCAGGAAGCCATTCAGAATTTTTCACCCT |
| BX647159 | BX647159 | AGGTTTTCTCTCTTGGTCCTACCTTGTGTATGTAGAAAGTTAATGTAAGCTTTTCTTCTG |
| NM_153256 | C10orf47 | AAGATCTCAGGGTAGGGAAAAACCAGTGAATGAGGTTACACAGCAGGAAAAGAACCGGTT |
| NM_018363 | C10orf59 | CCATTTGGAGTTACATACTTGGAACACAGCATTGAGGATGTGCAAGAGTTAGTCTTCCAG |
| NM_025113 | C13orfl8 | TGTGTCAAGGAAAGGGCTTTATTTGTGAATTTTGCCAGAATACGACTGTCATCTTCCCAT |
| NM_013399 | C16orf5 | GTGAATGATCAGACCCTGGTAGCTAAGAAGGAACTTGTCCCTTTGAGTCAGTGTGCAGAC |
| NM_016470 | C20orf111 | TACATGGAGTATTACTTGTATATTCCCAAGAAAATGTCCCACATGGCAGAAATGATGTAC |
| NM_001080472 | C20orf142 | TCTGGGTGTTGATGTTTCTGTGCACAGCTGTTTATTTCCACAACTTGTCCCAGAAGGTGT |
| BC029662 | C20orf142 | TTGTATTTAGAGAATGGCTCTTGCCACTGTTATGTACACACAGATCGTGTGTGTAAACAA |
| NM_015511 | C20orf4 | TTCCTCTCTAGACAAGTACACAGGCCTGCCACCTGACATCAAACTGTTGTACTATGATCA |
| NM_080748 | C20orf52 | ACTACATCTGTCCCTTCCCATCAATCCCAGCCCATGTACTAATAAAAGAAAGTCTTTGAG |
| NM_001218 | CA12 | GTTCCCCGAGAACTTTCTGATTCACAGTCTCATTTTGACAGCATGAAATGTCCTCTTGAA |
| NM_000719 | CACNA1C | GAGTTTTACCAACCATTGTGTATGCCCAATAATTTGTTATCATTTCCTTAGGTAGTAACC |
| NM_004929 | CALB1 | CTGATTCTATGCCTGTATTTCCAACAAACTTACTGTCAGAGAATATGACCTAAATCCATT |
| NM_032587 | CARD6 | ACTAGCTTCTGCTAATAGCCCCAATTTGCTTGAATGGGAAAACTCTCTCATTTGACCCTT |
| NM_004364 | CEBPA | GCCTTGTTTGTACTGTATGCCTTCAGCATTGCCTAGGAACACGAAGCACGATCAGTCCAT |
| NM_015147 | CEP68 | TTACTTTCTTGGCTAACCAGTTTCTTAGAAGAAAATGTGTCAGGGACTTGGGGATCTACA |
| AK098629 | CNNM3 | AGACAGTGTGGTCTGTTGACAGTATGGAATATTCTTTACCCATCAGATATGATCTGTATG |
| CR616309 | CR616309 | GCCCCATTTCAAGTATAACCAGGAGGGAAAATGGTGCTTGAAATAAGCATGCCACAAAGG |
| NM_030877 | CTNNBL1 | CAGGAAGATCAAGAAAGTGGGAACCACTGAGAAGGAACATGAAGAGCATGTCTGTTCGAT |
| NM_000784 | CYP27A1 | AGCTATTTGCTACATCCTGTTCGAGAAACGCATTGGCTGCCTGCAGCGATCCATCCCCGA |
| NM_033081 | DIDO1 | ATATGATCTTAAGAGTCTAAACATTCAAGAGACGAGGGCAAGAAAGCCAGTCACATGTAG |
| AL390175 | DKFZp547K054 | AAAGAATATCTGAGACGACGTTTGGGAGCAATGCTCTTTACCACGTTCACCAAGTCGTCT |
| NM_012242 | DKK1 | AATAACACCTTCCAAAAACCTGGAGTGTAAGAGCTTTGTTTCTTTATGGAACTCCCCTGT |
| XM_085578 | DNAH2 | GCCGGACCTCACTCAGACTTTGACCTTGGCCGAATTTGTGTGATGTGGCCCTGGAGATAC |
| NM_052951 | DNTTIP1 | ATCTGAAGCTAGAGGAATTGAAATCCTTTGTCCTACCCTCCTGGATGGTGGAGAAGATGA |
| NM_152511 | DUSP18 | CTCCTTTCTGTGCACAGCACTTTATTGTTACAAAGTACTCTTCCAAAAAGTTACCCTGTG |
| NM_001946 | DUSP6 | GCTTGTGTTGTCGCAAAGGGGATAATCTGGGAAAGACACCAAATCATGGGCTCACTTTAA |
| NM_014183 | DYNLRB1 | ACACTGAAGCGACTGCAGAGCCAGAAGGGAGTGCAGGGAATCATCGTCGTGAACACAGAA |
| NM_207032 | EDN3 | AAGTTATTACAGGTATAAAAGTGATGACCTATCATGAGGAAATGAAAGTGGCTGATTTGC |
| NM_002212 | EIF6 | TCACCTTCCAAGTTGTTCCATGGGCTCCTGGCTCTGGACTGTGGCCAACCTTCTCCACAT |
| NM_006208 | ENPP1 | CCATCATAGGAATGGGCATTTGAACTTTGAAACTGAATGTGGTGATTACACTTCATGCTG |
| NM_017549 | EPDR1 | TCTAGATGCTTCTACTGTTATGTTTTATCTGCCCATTTATCTTTCTTAGTTACCAGGAGA |
| NM_004454 | ETV5 | TTTATAGCTTTCTTAACCTACACATAGTCTATACATAGATGCATATTTTACCCCCAGCTG |
| NM_021727 | FADS3 | AACTTTGAAGTGGAAAATCTGGCGTACATGCTGGTGTGCATGCAGTGGGCGGATTTGCTC |
| CR936791 | FAM169A | AGAAAGGCTTACCTTCTGTCATCAAGTGATTGTATCATCCTGGATCGTCATTTCCAAGGA |
| NM_001452 | FOXF2 | TGTGGGATCTTCGTTGCCTTCAGTAATCAGGGTGTGAAAAAAGCAGACAAGTTGTGTGTG |
| NM_178026 | GGT7 | TGGCCCCGAGCTTAGGGATGTGCTTGCAAACCCTTCTCAAGGGTCTCACAACCCCAACAT |
| NM_015101 | GLT25D2 | AGCATTTTAGACTAGGACTGTTCTACTGTGAAGAAAGTTCTGTCTCCTTTAGCCCGGTTT |
| ENST00000371081 | GNAS | TAATATCCCTAGATGATGGCTACAGTTTCTCATCTTCCCGGCTATGTCCTATCACAATTT |
| NM_001098722 | GNG4 | CAAACTCCATCCAGTACATTCTTTCTTCTTTCATGAAAGAGCTTGAGTTGGATGTAAATA |
| NM_000273 | GPR143 | ATATTCCTCAGACTCAACAATTCTTGTTCTTTAGAACTGTGTTCTCACCTTCCCAACACT |
| NM_001030004 | HNF4A | CATGAAGAAGGAAGCCGTCCAGAATGAGCGGGACCGGATCAGCACTCGAAGGTCAAGCTA |
| NM_002147 | HOXB5 | AAATGTCTTGTGTATTTCTCTGTGAATCCATGGGTCTGGCTAGAGGGCCCAAAGCTTGTA |
| NM_018952 | HOXB6 | ATTAATTTTCTATGTGTTGTGTTTGTAGTCTTGTCTTAGCTCTGGACGTGAAATACTTCG |
| NM_024016 | HOXB8 | GCTGTGGGCAATTGTTACAAGTGTTCTTAGGTTTACTGTGAAGAGAATGTATTCTGTATC |
| NM_024501 | HOXD1 | TTTTTTCTTTAAAAAAGCGGTTTCTACCTCTCTATGTGCCTGAGTGATGATACAATCGCT |
| NM_014278 | HSPA4L | AGCTTTCCGGATAATTTTATATATCAAACATACAGGATGGATACATAGTTGGCAACAGTC |
| NM_021258 | IL22RA1 | ACCTACCTCATGGAGATTGTGGTGAAGATGAAATGAAGTCATGTCTTTAAAGTGCTTAAT |
| NM_033439 | IL33 | AACTTCATTTCTGTATCACCTGACCTCTGGATGCCAAAACGTTTATTCTGCTTTGTCTGT |
| THC2657554 | KCNK5 | CTGTCTCCAGGTAGGTGGACCAGAGAACTTGAGCGAAGCTCAAGCCTTCTCAACTCAAGG |
| NM_003740 | KCNK5 | CTGTGAAATGTTTTAATGAACCATGTTGTTGCTGGTTGTCCTGGCATCGCGCACACTGTA |
| NM_198991 | KCTD1 | TTGATGGTACAGAGCCCATTGTTTTGGACAGTCTCAAACAGCACTATTTCATTGACAGAG |
| NM_018689 | KIAA1199 | AACGCTCCTCTGAAATGCTTGTCTTTTTTCTGTTGCCGAAATAGCTGGTCCTTTTTCGGG |
| NM_018689 | KIAA1199 | TGTTTCACAGTACAGGATCTGTACATAAAAGTTTCTTTCCTAAACCATTCACCAAGAGCC |
| NM_002776 | KLK10 | GCTGTCTACACCCAGATCTGCAAATACATGTCCTGGATCAATAAAGTCATACGCTCCAAC |
| NM_002776 | KLK10 | TGCCTACCTCTTAGACATGTTGTGAGGAGACTATGATATAACATGTGTATGTAAATCTTC |
| NM_144947 | KLK11 | TCCTGGACTACAGGAGATGCTGTCACTTAATAATCAACCTGGGGTTCGAAATCAGTGAGA |
| NM_019079 | L1TD1 | TTCTACCCAGAAGGATGGACAGCTAATAGCGTACTTGGGGATGAGGAGCAAGGAATATTA |
| NM_001001552 | LEMD1 | TCTACCTGACTGTGGAAAATAAGTCGCTGTTTGGTTAAGTAATTTAGGAGCAAAGCAATG |
| XM_001715202 | LOC100130910 | CAACTTTTAAACATTGGTTACCTTTTGGGAGAATAAAGGGAATGGGGGCGATGATCACAG |
| ENST00000398035 | LOC120376 | CACAGTTTACATGTCACTATTTCCAATTTTCTGATGCTAAGCATTCATATGAAGTCCTCA |
| AK025743 | LOC157860 | GGGGTTTAGGGTCGAGCTGTTCCTGATGTTTATCGGAGACTGGGATCAAAGCTATCCAGG |
| NM_001013642 | LOC388610 | GGTGGGGGACACACCTTTTTCTCAGGAAGAGGTGATGGCAATGTAAAACATCTAAGCAAA |
| THC2688670 | LOC646951 | CCTGGGTTGTTGTGCATATTAGGACGTAGTATGTGTGTTGAAACGCCCCATACAGAATGA |
| NM_000239 | LYZ | GATTATGGGATATTTCAGATCAATAGCCGCTACTGGTGTAATGATGGCAAAACCCCAGGA |
| NM_000249 | MLH1 | TGTGGGATGTGTTCTTCTTTCTCTGTATTCCGATACAAAGTGTTGTATCAAAGTGTGATA |
| NM_014484 | MOCS3 | ATTTGCAAACTGGGAAATGACTCACAGAAAGCCGTGAAGATCCTCCAGTCCTTATCAGCA |
| NM_024657 | MORC4 | CACCACACCAGAAATGGGACCTAAATTAAGGCTTAAAGAATGAGCATGGCCACACTGTCA |
| NM_000260 | MYO7A | ATACCAACTGGGCCTCTGATGTTCTTCCAGTGAGGCATCTCTCTGGGATGCAGAACTTCC |
| THC2669157 | OIT3 | CAGCAAATGTACTAATTTACACTGTCACTGTATACTTCTCCCTATAACCTTGTAAGTGTT |
| NM_033100 | PCDH21 | TTCAGCAGGTGGATCCATTCTTCGACCCCCAGATGTGACTCTAAAGAAGGCTGAAAATTT |
| NM_007350 | PHLDA1 | TACAGGATCTAAAAGGGTTTTCTTAGAAAGGGCAATATTGTCCAATGAAGTAAGCAGAAG |
| NM_080476 | PIGU | CGGACCCCTATGGAAATGCGTTACATCCCTTTGAAAGTGGCCCTGTTCTATCTCTTAAAT |
| NM_032562 | PLA2G12B | CATCTGTGCAGAGGAGGAGAAGGAAGAGTTATGAGGAAGAAGTGATTCCTTCCTGGTTTT |
| NM_002657 | PLAGL2 | AGAGAAAAGTACAAGACAGAAATCTTCTAGCACTTTGTAAACACAGTGAATAACCTCTTG |
| NM_025225 | PNPLA3 | CTTGACTACTAAAAACGTCTCCATGGCGGGGGTAACAAGATGATAATCTACTTAATTTTA |
| NM_015352 | POFUT1 | ACTGGGAATACCGCAGTGAATAAGATAAACTCCGTGTCCTTGTAGAGCCTTCATTTTAGT |
| NM_172236 | POFUT1 | GGATCAGTTTCATGTGAGTTTCAACAAGTCGGAGCTTTTTACAGGCATTTCCTTCAGTGC |
| NM_015352 | POFUT1 | TTCACTATTGTTATTTGAAAAAATATTTTTATTTGTAAGAGTTTTTCTTTATTTAAAATG |
| NM_012094 | PRDX5 | ATCTTTGGGAATCGACGTCTCAAGAGGTTCTCCATGGTGGTACAGGATGGCATAGTGAAG |
| NM_080685 | PTPN13 | GCAGATCTTAGGGATGATTAAAGGCAGCATTTGATGATAGCAGACATTGTTACAAGGACA |
| NM_030667 | PTPRO | AACTGTTCTAGTTCTTGATGGTTTTCTTCCTTATTAACAGTTGGTGTTTCTTCCTTGGCC |
| NM_007238 | PXMP4 | CCCAAAGGATACTGCCTTCTCAAGATCATAGGCCTCAGACTCCAACTGGTGTTATCCCAG |
| NM_014298 | QPRT | GCCCTTGATTTCTCCCTCAAGCTGTTTGCCAAAGAGGTGGCTCCAGTGCCCAAAATCCAC |
| NM_004163 | RAB27B | ATGTATCTGCTAGACTCTACATAGAAACTGAACATCAAGAACCCCACCAAAATATTACTT |
| NM_006834 | RAB32 | ACCAAAGCTTTCCTAATGAAGAAAACGATGTGGACAAAATTAAGCTAGATCAAGAGACCT |
| NM_032044 | REG4 | CTCTTCCTTTCTGCTAGCCTGGCTAAATCTGCTCATTATTTCAGAGGGGAAACCTAGCAA |
| NM_014899 | RHOBTB3 | ACACATGTATAAAGATGTTCACAGAGAAAGATGCTCTGTAGAGAATTTGCTACCGAAGTT |
| NM_198232 | RNASE1 | GTTAGGGCTCCTATTCAACACACACATGCTTCCCTTTCCTGAGTCCCATCCCTGCGTGAT |
| NM_017763 | RNF43 | GGCAGAATTACAGCTGAGCGGGGACAACAAAGAGTTCTTCTCTGGGAAAAGTTTTGTCTT |
| NM_153225 | RPESP | TCCTCTCATTCAGCAGGTGGCCCGTGAGACACAGAATACATGTCTGTTTGCTAAAGTAAA |
| NM_005980 | S100P | CCAAAAGTGTTTGTTGGCAATTATTCCCCTAGGCTGAGCCTGCTCATGTACCTCTGATTA |
| NM_002639 | SERPINB5 | CATCAGGCACAACAAAACTCGAAACATCATTTTCTTTGGCAAATTCTGTTCTCCTTAAGT |
| NM_001649 | SHROOM2 | GTGGTCATTTTGATGATATGTGTGTAAAATGTGAATAATCCAATTGGTGTCTGTACTCAG |
| NM_020717 | SHROOM4 | TGCCAGGTTTAACCACTCATAGCAACAAGACTTTTACCCAGAGACCAAAACCTATAGACC |
| NM_003059 | SLC22A4 | AAACAAGAGACTCAATGGAGACAGAAGAAAATCCCAAGGTTCTAATAACTGCATTCTGAA |
| NM_000112 | SLC26A2 | TCCTCCTTTGAAGCTAATGGCATTTGTATATACACACTGCAGCAGAGCTTGTAGCTGGAC |
| NM_173596 | SLC39A5 | TCACGTGGATGGTCCTCCTGGGAGATGGTCTACACAACCTCACTGATGGGCTGGCCATAG |
| NM_021095 | SLC5A6 | TTTTTCTCGTCGCTTGCCAATCTGTTTTTTAAAGGATCAGGCTCGTAGGGAGCAGGATCA |
| NM_014331 | SLC7A11 | CATTTTGCTTTCCTAACCATTCAGTCAGGAATTAAAATATGGCATTGTATAACAACTGGG |
| NM_019844 | SLCO1B3 | TCCTGGTCTTTCACTGACAATTCCAACATTCTTTACTTACAGTGGACCAATGGATAAGTC |
| NM_032229 | SLITRK6 | TCCATACCTTGTAAATAAGTGCCTTACGTGAGTGTGTCATCAATCAGAACCTAAGCACAG |
| NM_019008 | SMCR7L | CAAGGATTTCTTATGGTGGTTTCAGTTTCATTTGCATAAAGGTATTGAGAGGGAACAAAA |
| NR_003239 | SNHG11 | CCTGGAGGGGCCTCCTCATGATTGTTTGGGGGTTGATCACAGACCAAGAGTGACGAGTGA |
| NM_003098 | SNTA1 | TCCTGCGACAGCCCTTCGAGAAGCTGCAGATGTCTTCAGATGACGGTGCCAGTCTCCTTT |
| NM_001042633 | SNX21 | CCATGATGAAGCCAAATCTGAACCCATGTCCTCACTTCAAGGTTATCAATCTTGGATTGT |
| NM_001034954 | SORBS1 | ACGTCGCCTTCCTATGTATGACGAAACAAGAAACAGAGATTTCCAATTGCTCTTTTGTCT |
| NM_001034954 | SORBS1 | TTGGTACTTCAAGAAGGACAAAGCAGTTTGGTACTTTTCCAGGCAACTATGTAAAACCTT |
| NM_014587 | SOX8 | CCTGCTGCCTTCACTCCTTTCTGACCAAGCAACGCTAACTTTTGTACAGATCGATTTGAT |
| NM_145263 | SPATA18 | ACTTGCTGTTTCTTCAGTTTCTAATTTGAGCTGTAACTACACAAGGAAAGCTAAATAGTC |
| NM_003122 | SPINK1 | GGAAACGCCAGACTTCTATCCTCATTCAAAAATCTGGGCCTTGCTGAGAACCAAGGTTTT |
| NM_006804 | STARD3 | AACTGGAAGTTCGAGAAGAATAATGAATATGGGGACACCGTGTACACCATTGAAGTTCCT |
| NM_001062 | TCN1 | CCAGAAAATGAATGATACTATATTTGGTTTCACAATGGAGGAGCGCTCATGGGGGCCCTA |
| NM_005423 | TFF2 | TCCAACTTCATCTTTGAAGTGCCCTGGTGCTTCTTCCCGAAGTCTGTGGAAGACTGCCAT |
| THC2624002 | THC2624002 | CCAGACTGATGAAATTCCTTCCAGTTTCAGAAAAGATTATCACCAATCCCTACATTATAA |
| THC2634713 | THC2634713 | GAACCCAGTGATGGAGCCTTAAGTAAAGAGTATAATTTTGGTGTGTGGTTGGTAGAATCA |
| THC2699065 | THC2699065 | GGTTTATTGACATGGAATGATTTGACCAAGGTGATATAGTGGTTAAGCAGTTAAGTGATT |
| THC2708669 | THC2708669 | GAGGGACTATAAAGGCCAAGACCTTGTTCTTGCCATTTTAGAGATTCAGAATATAATCTA |
| NM_138554 | TLR4 | TTACTGAGTGTTTCAGAGTGTGTTTGGTTTGAGCAGGTCTAGGGTGATTGAACATCCCTG |
| NM_144649 | TMEM71 | AACCACGTTCTAAACCAAGTTTTTAATCTTCTTGGGGCTCGTAATTACCTTTCACTTTAA |
| BE893137 | TNIK | AGCAAGATATCCTCCTCATGGTCCCTTTAGCTCTCAAAAGCAATGAAATCCTCCTGTTCT |
| NM_003279 | TNNC2 | GACTTCGACGAGTTCCTGAAGATGATGGAGGACGTGCAGTAAGGAGTGGACAGTCGCCTC |
| BC107798 | TNNT1 | TCCGAGCGTAAGAAGCCTCTGGACATTGACTACATGGGGGAGGAACAGCTCCGGGAGAAA |
| NM_022748 | TNS3 | GCTGGTATAGGAAGTTTCTTTCTTCCTTTTGTGTTACATGTGCATTAAACAGAACAAGCT |
| NM_032865 | TNS4 | GGGCTGGCAGAGGCCCCCATATACTCTGCTACAGCTCCTATCATGAAAAATAAAATGTTT |
| NM_021202 | TP53INP2 | GGGGGAAGAGTTTAAGTTATAGGGCATTTGGCTCAAATTTTAAAAGGCCTTTTGTTTACC |
| NM_203297 | TRIM7 | AGCTCTGCCTTGAGAGGTTGTGGGACCTCTGGATCCAGCTGACCTGACAGGTCATCTACT |
| NM_033342 | TRIM7 | CGTAACATACCAGTTAGGGCCTGCGGAAGCATCTTGTAATGGAACACATTACTATTTCTG |
| NM_130783 | TSPAN18 | TGCTCTGGCAACCCCATAAATGACACCTGAGCTCCGTAGAAGCTAAGCTCCTGAGACCCA |
| NM_023076 | UNKL | AGCAGAGGGGATCCAACGTCAGAGCTTTTAGAATTACTTTTTTAAGCAGCTGTCTTCTGG |
| Unknown | Unknown | GCACCAGGGGGAGGATTTTCATCAAGCACCCACACCTCTTTAAGTTTGCAGCAGATCCTC |
| NM_006113 | VAV3 | TTGCCAACCCTGGTATGCTGGAGCAATGGAAAGATTGCAAGCAGAGACCGAACTTATTAA |
| NM_021197 | WFDC1 | AAGCAGCTAGGTTGCAAGAACATTCCTCTACTTTCTGCTAAGCCTTGGAAACAGTTGGGA |
| NM_013313 | YPEL1 | GGAGTAATGTGCGAACTTTCCCTTCTCCTTCGAATGCTGTTTTGTGAAAGAAACTGTGAA |
| NM_033132 | ZIC5 | GTCTTCAGATTGTTGTGATGAATATGAAACATGCAGACCCTCCTTTATAAAGAAAAAGAC |
| NM_080752 | ZSWIM3 | TGTGAGAGTTTAAAGTGGGCAGGACATACTAGGGTTTAGCATTTTAGCCAATGTCTTCCT |

## Claims

1. A method for typing a RNA sample of an individual suffering from cancer, or suspected of suffering there from, the method comprising
a. providing an RNA sample that is prepared from a tissue sample from said individual, said tissue sample comprising colorectal cancer cells or suspected to comprise colorectal cancer cells;
b. determining RNA levels for a set of at least five genes selected from TRIM7, ACSL6, AGR2, AHR, AIFM3, ANXA10, ARID3A, ASCL2, ASXL1, AXIN2, BX427767, BX647159, C10orf47, C10orf59, C13orf18, C20orf142, CEBPA, CEP68, CR616309, DIDO1, DNAH2, DUSP18, EPDR1, ETV5, GGT7, GPR143, HOXD1, KCNK5, LOC157860, LOC388610, MLH1, MOCS3, MORC4, PCDH21, PLAGL2, POFUT1, PRDX5, PTPRO, QPRT, RAB27B, RAB32, REG4, RNF43, SHROOM2, SHROOM4, SLC39A5, SLC7A11, SMCR7L, SNHG11, SOX8, SPINK1, TFF2, TNNC2, TNNT1, UNKL, VAV3, ZIC5 and ZSWIM3 listed in Table 2 in said RNA sample, and
c. typing said RNA sample on the basis of the RNA levels determined for said set of genes.

2. The method of claim 1, wherein said set of genes comprises a total of ten genes listed in Table 2.

3. The method of claim 1, wherein said set of genes comprises all genes listed in Table 2.

4. The method according to any of claims 1-3, whereby said RNA levels are determined by microarray analysis.

5. The method according to any of the previous claims, further comprising normalizing the determined RNA levels of said set of genes in said sample.

6. The method according to any of the previous claims, whereby said typing differentiates cancer cells with one or more activating mutations in an BRAF pathway from cancer cells without an activating mutation in said BRAF pathway.

7. A method of classifying an individual suffering from cancer, comprising: classifying said individual as having one or more activating mutations in the BRAF pathway or not having an activating mutation in the BRAF pathway by a method comprising:
(a) providing an RNA sample from a said individual that is prepared from a tissue sample from said individual, said tissue sample comprising colorectal cancer cells or suspected to comprise colorectal cancer cells;
(b) determining a level of RNA for a set of genes comprising at least five of the genes TRIM7, ACSL6, AGR2, AHR, AIFM3, ANXA10, ARID3A, ASCL2, ASXL1, AXIN2, BX427767, BX647159, C10orf47, C10orf59, C13orf18, C20orf142, CEBPA, CEP68, CR616309, DIDO1, DNAH2, DUSP18, EPDR1, ETV5, GGT7, GPR143, HOXD1, KCNK5, LOC157860, LOC388610, MLH1, MOCS3, MORC4, PCDH21, PLAGL2, POFUT1, PRDX5, PTPRO, QPRT, RAB27B, TRIM7, RAB32, REG4, RNF43, SHROOM2, SHROOM4, SLC39A5, SLC7A11, SMCR7L, SNHG11, SOX8, SPINK1, TFF2, TNNC2, TNNT1, UNKL, VAV3, ZIC5 and ZSWIM3 listed in Table 2 in said sample;
(c) determining a first similarity value between a level of expression from the set of genes in said individual and a level of expression from said set of genes in profile template (gene signature) that is representative of samples not having an activating mutation in the BRAF pathway;
(d) determining a second similarity value between a level of expression from the set of genes in said individual and a level of expression from said set of genes in profile template (gene signature) that is representative of samples having an activating mutation in the BRAF pathway;
(e) determining a difference between the first similarity and the second similarity; and
(f) classifying said individual as having an activating mutation in the BRAF pathway if said difference value is below a similarity threshold value, and classifying said individual as not having an activating mutation in the BRAF pathway if said similarity value exceeds said similarity threshold value.

8. The method of claim 7, whereby only one profile template is used for typing a sample.

9. The method of claim 7 or claim 8, whereby said set of genes comprises all genes listed in Table 2.

10. The method of claim 7 or claim 8 or claim 9, whereby normalized levels of RNA are used to determine a similarity value.

11. Small molecule and/or antibody for use in a method of treating an individual suffering from cancer with anti-BRAF therapy, the method comprising
determining whether a level of expression of at least five genes of TRIM7, ACSL6, AGR2, AHR, AIFM3, ANXA10, ARID3A, ASCL2, ASXL1, AXIN2, BX427767, BX647159, C10orf47, C10orf59, C13orf18, C20orf142, CEBPA, CEP68, CR616309, DIDO1, DNAH2, DUSP18, EPDR1, ETV5, GGT7, GPR143, HOXD1, KCNK5, LOC157860, LOC388610, MLH1, MOCS3, MORC4, PCDH21, PLAGL2, POFUT1, PRDX5, PTPRO, QPRT, RAB27B, TRIM7, RAB32, REG4, RNF43, SHROOM2, SHROOM4, SLC39A5, SLC7A11, SMCR7L, SNHG11, SOX8, SPINK1, TFF2, TNNC2, TNNT1, UNKL, VAV3, ZIC5 and ZSWIM3 listed in Table 2in a tissue sample from said individual correlates with the level of these genes in a reference sample having an activating mutation in the BRAF pathway according to any one of claims 7-10, whereby the set of genes comprises TRIM7, and whereby said tissue sample comprising colorectal cancer cells or suspected to comprise colorectal cancer cells, and
selecting an individual in which the level of expression of the genes correlates with the level of expression in the reference sample for treatment with anti-BRAF therapy .

12. Small molecule and/or antibody for use according to claim 11, wherein said small molecule is PLX4032.

## Patentansprüche

1. Verfahren zum Typisieren einer RNA-Probe eines Individuums, leidend an Krebs, oder im Verdacht stehend, daran zu leiden, das Verfahren umfassend
a. Bereitstellen einer RNA-Probe, die aus einer Gewebeprobe des Individuums hergestellt wird, die Gewebeprobe umfassend Darmkrebszellen oder im Verdacht stehend, Darmkrebszellen zu umfassen;
b. Bestimmen der RNA-Spiegel für einen Satz von wenigstens fünf Genen, ausgewählt aus TRIM7, ACSL6, AGR2, AHR, AIFM3, ANXA10, ARID3A, ASCL2, ASXL1, AXIN2, BX427767, BX647159, C10orf47, C10orf59, C13orf18, C20orf142, CEBPA, CEP68, CR616309, DIDO1, DNAH2, DUSP18, EPDR1, ETV5, GGT7, GPR143, HOXD1, KCNK5, LOC157860, LOC388610, MLH1, MOCS3, MORC4, PCDH21, PLAGL2, POFUT1, PRDX5, PTPRO, QPRT, RAB27B, TRIM7, RAB32, REG4, RNF43, SHROOM2, SHROOM4, SLC39A5, SLC7A11, SMCR7L, SNHG11, SOX8, SPINK1, TFF2, TNNC2, TNNT1, UNKL, VAV3, ZIC5 und ZSWIM3, gelistet in Tabelle 2, in der RNA-Probe, und
c. Typisieren der RNA-Probe auf der Grundlage der RNA-Spiegel, bestimmt für den Satz von Genen.

2. Verfahren nach Anspruch 1, wobei der Satz von Genen eine Gesamtzahl von zehn Genen, gelistet in Tabelle 2, umfasst.

3. Verfahren nach Anspruch 1, wobei der Satz von Genen alle Gene, gelistet in Tabelle 2, umfasst.

4. Verfahren nach einem der Ansprüche 1 - 3, wodurch die RNA-Spiegel durch Microarray-Analyse bestimmt werden.

5. Verfahren nach einem der vorhergehenden Ansprüche, ferner umfassend Normalisieren der bestimmten RNA-Spiegel des Satzes von Genen in der Probe.

6. Verfahren nach einem der vorhergehenden Ansprüche, wodurch das Typisieren Krebszellen mit einer oder mehreren aktivierenden Mutationen in einem BRAF-Signalweg von Krebszellen ohne eine aktivierende Mutation in dem BRAF-Signalweg unterscheidet.

7. Verfahren zum Klassifizieren eines Individuums, leidend an Krebs, umfassend: Klassifizieren des Individuums als aufweisend eine oder mehrere aktivierende Mutationen in dem BRAF-Signalweg oder nicht-aufweisend eine aktivierende Mutation in dem BRAF-Signalweg durch ein Verfahren, umfassend:
(a) Bereitstellen einer RNA-Probe von dem Individuum, die aus einer Gewebeprobe von dem Individuum hergestellt wird, die Gewebeprobe umfassend Darmkrebszellen oder im Verdacht stehend, Darmkrebszellen zu umfassen;
(b) Bestimmen eines RNA-Spiegels für einen Satz von Genen, umfassend wenigstens fünf der Gene TRIM7, ACSL6, AGR2, AHR, AIFM3, ANXA10, ARID3A, ASCL2, ASXL1, AXIN2, BX427767, BX647159, C10orf47, C10orf59, C13orf18, C20orf142, CEBPA, CEP68, CR616309, DIDO1, DNAH2, DUSP18, EPDR1, ETV5, GGT7, GPR143, HOXD1, KCNK5, LOC157860, LOC388610, MLH1, MOCS3, MORC4, PCDH21, PLAGL2, POFUT1, PRDX5, PTPRO, QPRT, RAB27B, TRIM7, RAB32, REG4, RNF43, SHROOM2, SHROOM4, SLC39A5, SLC7A11, SMCR7L, SNHG11, SOX8, SPINK1, TFF2, TNNC2, TNNT1, UNKL, VAV3, ZIC5 und ZSWIM3, gelistet in Tabelle 2, in der Probe;
(c) Bestimmen eines ersten Ähnlichkeitswerts zwischen einem Expressionsspiegel aus dem Satz von Genen in dem Individuum und einem Expressionsspiegel aus dem Satz von Genen in der Profilvorlage (Gensignatur), die repräsentativ für Proben ist, nicht aufweisend eine aktivierende Mutation in dem BRAF-Signalweg;
(d) Bestimmen eines zweiten Ähnlichkeitswerts zwischen einem Expressionsspiegel aus dem Satz von Genen in dem Individuum und einem Expressionsspiegel aus dem Satz von Genen in der Profilvorlage (Gensignatur), die repräsentativ für Proben ist, aufweisend eine aktivierende Mutation in dem BRAF-Signalweg;
(e) Bestimmen einer Differenz zwischen der ersten Ähnlichkeit und der zweiten Ähnlichkeit; und
(f) Klassifizieren des Individuums als aufweisend eine aktivierende Mutation in dem BRAF-Signalweg, wenn der Differenzwert unter einem Ähnlichkeitsschwellenwert ist, und Klassifizieren des Individuums als nicht-aufweisend eine aktivierende Mutation in dem BRAF-Signalweg, wenn der Ähnlichkeitswert den Ähnlichkeitsschwellenwert überschreitet.

8. Verfahren nach Anspruch 7, wodurch nur eine Profilvorlage zum Typisieren einer Probe verwendet wird.

9. Verfahren nach Anspruch 7 oder Anspruch 8, wodurch der Satz von Genen alle Gene, gelistet in Tabelle 2, umfasst.

10. Verfahren nach Anspruch 7 oder Anspruch 8 oder Anspruch 9, wodurch normalisierte Spiegel von RNA verwendet werden, um einen Ähnlichkeitswert zu bestimmen.

11. Kleines Molekül und/oder Antikörper zur Verwendung in einem Verfahren zum Behandeln eines Individuums, leidend an Krebs, mit Anti-BRAF-Therapie das Verfahren umfassend Bestimmen, ob ein Expressionsspiegel von wenigstens fünf Genen von TRIM7, ACSL6, AGR2, AHR, AIFM3, ANXA10, ARID3A, ASCL2, ASXL1, AXIN2, BX427767, BX647159, C10orf47, C10orf59, C13orf18, C20orf142, CEBPA, CEP68, CR616309, DIDO1, DNAH2, DUSP18, EPDR1, ETV5, GGT7, GPR143, HOXD1, KCNK5, LOC157860, LOC388610, MLH1, MOCS3, MORC4, PCDH21, PLAGL2, POFUT1, PRDX5, PTPRO, QPRT, RAB27B, TRIM7, RAB32, REG4, RNF43, SHROOM2, SHROOM4, SLC39A5, SLC7A11, SMCR7L, SNHG11, SOX8, SPINK1, TFF2, TNNC2, TNNT1, UNKL, VAV3, ZIC5 und ZSWIM3, gelistet in Tabelle 2, in einer Gewebeprobe des Individuums mit dem Spiegel dieser Gene in einer Referenzprobe, aufweisend eine aktivierende Mutation in dem BRAF-Signalweg nach einem der Ansprüche 7 - 10, korreliert, wodurch der Satz von Genen TRIM7 umfasst, und wodurch die Gewebeprobe umfassend Darmkrebszellen oder im Verdacht stehend, Darmkrebszellen zu umfassen, und
Auswählen eines Individuums, bei dem der Expressionsspiegel der Gene mit dem Expressionsspiegel in der Referenzprobe für Behandlung mit Anti-BRAF-Therapie korreliert.

12. Kleines Molekül und/oder Antikörper zur Verwendung nach Anspruch 11, wobei das kleine Molekül PLX4032 ist.

## Revendications

1. Procédé pour typer un échantillon d'ARN d'un individu souffrant d'un cancer, ou suspecté d'en souffrir, le procédé comprenant
a. l'obtention d'un échantillon d'ARN qui est préparé à partir d'un échantillon tissulaire provenant dudit individu, ledit échantillon tissulaire comprenant des cellules de cancer colorectal ou étant suspecté de comprendre des cellules de cancer colorectal ;
b. la détermination des niveaux d'ARN pour un jeu d'au moins cinq gènes choisis parmi TRIM7, ACSL6, AGR2, AHR, AIFM3, ANXA10, ARID3A, ASCL2, ASXL1, AXIN2, BX427767, BX647159, C10orf47, C10orf59, C13orf18, C20orf142, CEBPA, CEP68, CR616309, DIDO1, DNAH2, DUSP18, EPDR1, ETV5, GGT7, GPR143, HOXD1, KCNK5, LOC157860, LOC388610, MLH1, MOCS3, MORC4, PCDH21, PLAGL2, POFUT1, PRDX5, PTPRO, QPRT, RAB27B, TRIM7, RAB32, REG4, RNF43, SHROOM2, SHROOM4, SLC39A5, SLC7A11, SMCR7L, SNHG11, SOX8, SPINK1, TFF2, TNNC2, TNNT1, UNKL, VAV3, ZIC5 et ZSWIM3 listés dans le Tableau 2 dans ledit échantillon d'ARN, et
c. le typage dudit échantillon d'ARN sur la base des niveaux d'ARN déterminés pour ledit jeu de gènes.

2. Procédé selon la revendication 1, dans lequel ledit jeu de gènes comprend un total de dix gènes listés dans le Tableau 2.

3. Procédé selon la revendication 1, dans lequel ledit jeu de gènes comprend tous les gènes listés dans le Tableau 2.

4. Procédé selon l'une quelconque des revendications 1 à 3, dans lequel lesdits niveaux d'ARN sont déterminés par analyse sur puce.

5. Procédé selon l'une quelconque des revendications précédentes, comprenant en outre la normalisation des niveaux d'ARN déterminés dudit jeu de gènes dans ledit échantillon.

6. Procédé selon l'une quelconque des revendications précédentes, dans lequel ledit typage différencie les cellules cancéreuses ayant une ou plusieurs mutations d'activation dans une voie de BRAF des cellules cancéreuses sans mutation d'activation dans ladite voie de BRAF.

7. Procédé de classification d'un individu souffrant d'un cancer, comprenant : la classification dudit individu comme ayant une ou plusieurs mutations d'activation dans la voie de BRAF on n'ayant pas de mutation d'activation dans la voie de BRAF par un procédé comprenant :
(a) l'obtention d'un échantillon d'ARN à partir dudit individu, qui est préparé à partir d'un échantillon tissulaire provenant dudit individu, ledit échantillon tissulaire comprenant des cellules de cancer colorectal ou étant suspecté de comprendre des cellules de cancer colorectal ;
(b) la détermination du niveau d'ARN pour un jeu de gènes comprenant au moins cinq des gènes TRIM7, ACSL6, AGR2, AHR, AIFM3, ANXA10, ARID3A, ASCL2, ASXL1, AXIN2, BX427767, BX647159, C10orf47, C10orf59, C13orf18, C20orf142, CEBPA, CEP68, CR616309, DIDO1, DNAH2, DUSP18, EPDR1, ETV5, GGT7, GPR143, HOXD1, KCNK5, LOC157860, LOC388610, MLH1, MOCS3, MORC4, PCDH21, PLAGL2, POFUT1, PRDX5, PTPRO, QPRT, RAB27B, TRIM7, RAB32, REG4, RNF43, SHROOM2, SHROOM4, SLC39A5, SLC7A11, SMCR7L, SNHG11, SOX8, SPINK1, TFF2, TNNC2, TNNT1, UNKL, VAV3, ZIC5 et ZSWIM3 listés dans le Tableau 2 dans ledit échantillon ;
(c) la détermination d'une première valeur de similarité entre le niveau d'expression pour le jeu de gènes dans ledit individu et le niveau d'expression pour ledit jeu de gènes dans une matrice de profils (signature génique) qui est représentative d'échantillons n'ayant pas de mutation d'activation dans la voie de BRAF ;
(d) la détermination d'une deuxième valeur de similarité entre le niveau d'expression pour le jeu de gènes dans ledit individu et le niveau d'expression pour ledit jeu de gènes dans une matrice de profils (signature génique) qui est représentative d'échantillons ayant une mutation d'activation dans la voie de BRAF ;
(e) la détermination d'une différence entre la première similarité et la deuxième similarité ; et
(f) la classification dudit individu comme ayant une mutation d'activation dans la voie de BRAF si ladite valeur de différence est inférieure à une valeur de seuil de similarité, et la classification dudit individu comme n'ayant pas une mutation d'activation dans la voie de BRAF si ladite valeur de similarité dépasse ladite valeur de seuil de similarité.

8. Procédé selon la revendication 7, dans lequel une seule matrice de profils est utilisée pour le typage d'un échantillon.

9. Procédé selon la revendication 7 ou la revendication 8, dans lequel ledit jeu de gènes comprend tous les gènes listés dans le Tableau 2.

10. Procédé selon la revendication 7 ou la revendication 8 ou la revendication 9, dans lequel des niveaux normalisés d'ARN sont utilisés pour la détermination d'une valeur de similarité.

11. Petite molécule et/ou anticorps pour une utilisation dans une méthode de traitement avec thérapie anti-BRAF d'un individu souffrant d'un cancer, le procédé comprenant
le fait de déterminer si le niveau d'expression d'au moins cinq gènes parmi TRIM7, ACSL6, AGR2, AHR, AIFM3, ANXA10, ARID3A, ASCL2, ASXL1, AXIN2, BX427767, BX647159, C10orf47, C10orf59, C13orf18, C20orf142, CEBPA, CEP68, CR616309, DIDO1, DNAH2, DUSP18, EPDR1, ETV5, GGT7, GPR143, HOXD1, KCNK5, LOC157860, LOC388610, MLH1, MOCS3, MORC4, PCDH21, PLAGL2, POFUT1, PRDX5, PTPRO, QPRT, RAB27B, TRIM7, RAB32, REG4, RNF43, SHROOM2, SHROOM4, SLC39A5, SLC7A11, SMCR7L, SNHG11, SOX8, SPINK1, TFF2, TNNC2, TNNT1, UNKL, VAV3, ZIC5 et ZSWIM3 listés dans le Tableau 2 dans un échantillon tissulaire provenant dudit individu est corrélé au niveau de ces gènes dans un échantillon de référence ayant une mutation d'activation dans la voie de BRAF selon l'une quelconque des revendications 7 à 10 moyennant quoi le jeu de gènes comprend TRIM7 et moyennant qoui ledit échantillon tissulaire comprend des cellules de cancer colorectal ou étant suspecté de comprendre des cellules de cancer colorectal, et
la sélection d'un individu chez lequel le niveau d'expression des gènes est corrélé avec le niveau d'expression dans l'échantillon de référence pour un traitement avec une thérapie anti-BRAF.

12. Petite molécule et/ou anticorps pour une utilisation selon la revendication 11, dans lequel ladite petite molécule est PLX4032.
